# EUROPEAN PATENT APPLICATION

(11) **EP 2 933 625 A1**
(43) Date of publication of application: **21.10.2015**
(21) Application number: 12889856.6
(22) Date of filing: 13.12.2012
(51) Int. Cl.: G01N 1/00, B01L 3/02, C12M 1/26

(54) **SUCTION TIP, OBJECT OBSERVATION DEVICE USING SAID SUCTION TIP, AND METHOD FOR OBSERVING OBJECT**

(71) Applicant: Yamaha Hatsudoki Kabushiki Kaisha (Yamaha Motor Co., Ltd.), Shizuoka 438-8501 (JP)
(72) Inventor: ITO, Saburo, Iwata-shi Shizuoka 438-8501 (JP)
(74) Representative: Müller-Boré & Partner Patentanwälte PartG mbB
(86) International application number: PCT/JP2012/007976
(87) International publication number: WO 2014/091525

(57) **Abstract**

Disclosed are a suction tip, an object observation device and an object observing method using the suction tip. The suction tip is provided with an internal tubular passage serving as a suction path for sucking an object, a distal end portion disposed in a substantially vertical direction when in use and including a suction port for sucking the object, the suction port being an opening formed in one end of the tubular passage; and a trap portion formed downstream of the distal end portion in a suction direction, and configured to trap the object to be sucked through the suction port. According to the suction tip, the object observation device and the object observing method using the suction tip, it is easy to collect the object, without falling and discharging of the collected object in the gravitational direction.

## Description

### TECHNICAL FIELD

The present invention relates to a suction tip, an object observation device and an object observing method using the suction tip, which enable to trap an object such as cells contained in a liquid while preventing the object from falling through a suction port even in a state that the suction port is immersed in the liquid after sucking the object.

### BACKGROUND ART

Conventionally, in various field, there are used suction pipettes configured to suck a relatively small quantity of liquid or the like by a predetermined volume for measurement. In particular, in biochemical experiments or the like, frequently used is a push-button type suction pipette, which is used by connecting a suction tip to a distal end of the pipette. A suction tip is a jig provided with a substantially cylindrical main body portion internally provided with a tubular passage serving as a suction path for sucking a liquid or the like. An opening formed in one end of the tubular passage functions as a suction port in sucking a liquid or the like, and a distal end of the suction pipette is inserted in an opening formed in the other end of the tubular passage, whereby the suction tip is mounted on the suction pipette.

A suction pipette is inherently configured to suck a liquid of a predetermined volume. However, as a preliminary usage, the suction pipette is used for the purpose of moving an object from a certain place to another place. In this case, the suction port of the suction tip is formed in a distal end of the suction tip so that the suction port comes sufficiently close to the object, and only the object is accurately sucked. Further, the object includes not only a liquid but also powders, particles, and cells derived from a living body contained in the liquid. In the case where the object is cells, operating the suction pipette connected to a suction tip, and screening the cells based on the shapes of the cells makes it possible to reduce a deviation of test conditions in various tests using the cells. The cells after screening and collection can be subjected to an HTS (High-Throughput Screening) process or a like process.

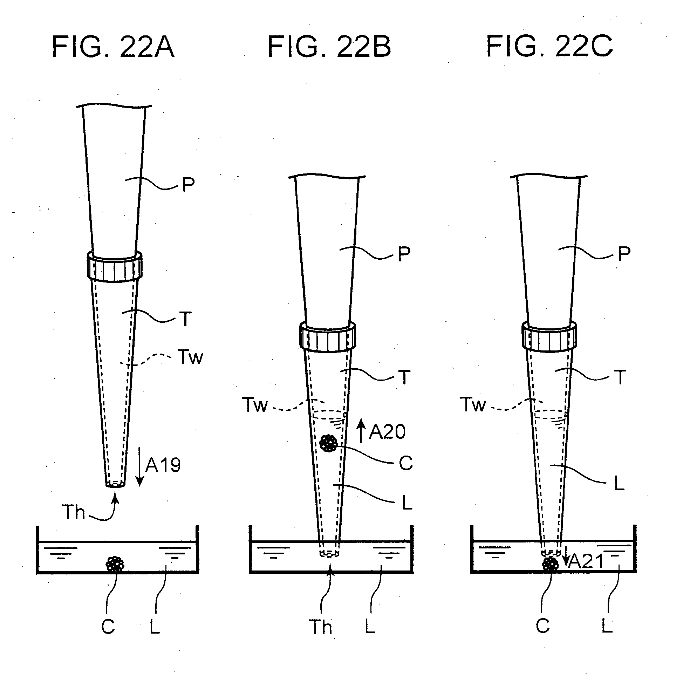

When cells held in a liquid stored in a vessel such as a Petri dish are sucked, the user operating the suction pipette holds the suction pipette in such a manner that the main body portion of the suction tip is set in an upright posture, immerses the suction port formed in the distal end of the suction tip in the liquid, and operates the suction pipette in a state that the suction port comes sufficiently close to the cells. FIG. 22A to FIG. 22C are schematic diagrams illustrating a manner as to how an object (cells C) is sucked using a suction pipette P connected to a conventional suction tip T. As illustrated in FIG. 22A, the user moves the suction pipette P close to the cells C. The arrow A19 indicates a moving direction of the suction pipette P. After the user moves a suction port Th close to the cells C, as illustrated in FIG. 22B, the user operates the suction pipette P to generate a suction force in a tubular passage Tw of the suction tip T, and sucks the cells C together with a liquid L around the cells C. The arrow A20 indicates a direction in which the cells C are sucked. Therefore, the sucked cells C sink in the liquid L within the tubular passage Tw in the gravitational direction. Thus, as illustrated in FIG. 22C, as far as the suction port Th is immersed in the liquid L, the cells C sink beyond the suction port Th, and are not collected. The arrow A21 indicates the sinking direction of the cells C which sink beyond the suction port Th. There is a suction tip configured such that a suction port is formed in a side of the suction tip (see Patent Literature 1). However, even in this configuration, a user cannot move the suction port to a position sufficiently close to cells that sink on an inner bottom portion of a vessel, and cannot efficiently collect the cells.

### CITATION LIST

### PATENT LITERATURE

Patent Literature 1: Japanese Unexamined Utility Model Publication No. H7-16163

### SUMMARY OF INVENTION

In view of the conventional problems as described above, an object of the invention is to provide a suction tip, an object observation device and an object observing method using the suction tip, which make it easy to collect an object held in a liquid, and which prevents the collected object from falling and discharging in the gravitational direction even in a state that a suction port is immersed in the liquid.

A suction tip according to an aspect of the invention is provided with an internal tubular passage serving as a suction path for sucking an object, a distal end portion disposed in a substantially vertical direction when in use and including a suction port for sucking the object, the suction port being an opening formed in one end of the tubular passage; and a trap portion formed downstream of the distal end portion in a suction direction, and configured to trap the object to be sucked through the suction port.

An object observation device according to another aspect of the invention is provided with a vessel including an inner bottom portion, and configured to store a liquid containing an object to be sucked; a suction tip having an internal tubular passage serving as a suction path for sucking the object, a distal end portion disposed in a substantially vertical direction when in use and including a suction port for sucking the object, the suction port being an opening formed in one end of the tubular passage, and a trap portion formed downstream of the distal end portion in a suction direction, and configured to trap the object to be sucked through the suction port; a suction means connected to the suction tip, and configured to generate a suction force for sucking the object; and an observing means provided with an optical lens system for capturing an image of the object to be trapped in the trap portion within a depth of field of the optical lens system.

An object observing method according to yet another aspect of the invention includes a sucking step of sucking an object by a suction means connected to a suction tip, the suction tip having an internal tubular passage serving as a suction path for sucking the object, a distal end portion disposed in a substantially vertical direction when in use and including a suction port for sucking the object, the suction port being an opening formed in one end of the tubular passage, and a trap portion formed downstream of the distal end portion in a suction direction, and configured to trap the object to be sucked through the suction port; a trapping step of trapping the sucked object in the trap portion; and an observing step of observing the trapped object.

These and other objects, features and advantages of the present invention will become more apparent upon reading the following detailed description along with the accompanying drawings.
FIG. 1 is a schematic diagram illustrating a configuration of a suction tip in a first embodiment of the invention;
FIG. 2 is a schematic diagram illustrating a behavior of a cell aggregate to be sucked using the suction tip in the first embodiment of the invention;
FIGS. 3A to 3C are schematic diagrams illustrating a behavior of a cell aggregate to be sucked using the suction tip in the first embodiment of the invention;
FIG. 4 is a schematic diagram illustrating a configuration of a suction tip in a second embodiment of the invention;
FIG. 5 is a schematic diagram illustrating a behavior of a cell aggregate to be sucked using the suction tip in the second embodiment of the invention;
FIGS. 6A to 6C are schematic diagrams illustrating a behavior of a cell aggregate to be sucked using the suction tip in the second embodiment of the invention;
FIG. 7 is a schematic diagram illustrating a configuration of a suction tip in a third embodiment of the invention;
FIG. 8 is a schematic diagram illustrating a behavior of a cell aggregate in the vicinity of a projection piece;
FIGS. 9A to 9C are schematic diagrams illustrating a behavior of a cell aggregate to be sucked using the suction tip in the third embodiment of the invention;
FIG. 10 is a schematic diagram illustrating a configuration of a suction tip in a fourth embodiment of the invention;
FIGS. 11A to 11C are schematic diagrams illustrating a behavior of a cell aggregate to be sucked using the suction tip in the fourth embodiment of the invention;
FIG. 12 is a schematic diagram illustrating a configuration of an object observation device in a fifth embodiment of the invention;
FIGS. 13A to 13D are schematic diagrams illustrating an operation of sucking and observing a cell aggregate using the object observation device in the fifth embodiment of the invention;
FIG. 14 is a flowchart illustrating the steps of an object observing method in a sixth embodiment of the invention;
FIG. 15 is a schematic diagram when the inventive suction tip is used as a jig to be connected to a conventional suction tip;
FIG. 16 is a schematic diagram illustrating a behavior of a plurality of cell aggregates to be sucked using another example of the suction tip in the first embodiment;
FIG. 17 is a schematic diagram illustrating another example of a converting portion provided in the suction tip in the second embodiment of the invention;
FIG. 18 is a schematic diagram illustrating another example of the converting portion provided in the suction tip in the second embodiment of the invention;
FIG. 19 is a schematic diagram illustrating another example of the projection piece provided in the suction tip in the third embodiment of the invention;
FIGS. 20A to 20D are schematic diagrams illustrating a behavior of a cell aggregate to be sucked using another example of the suction tip in the fourth embodiment of the invention;
FIGS. 21A and 21B are schematic diagrams illustrating another example of the suction tip in the first embodiment of the invention, specifically, schematic diagrams illustrating a suction tip provided with a spiral portion including a spirally curved tubular passage;
FIGS. 22A to 22C are schematic diagrams illustrating a manner as to how an object is sucked using a suction pipette connected to a conventional suction tip;
FIG. 23 is a microphotograph of a cell aggregate having a distorted shape; and

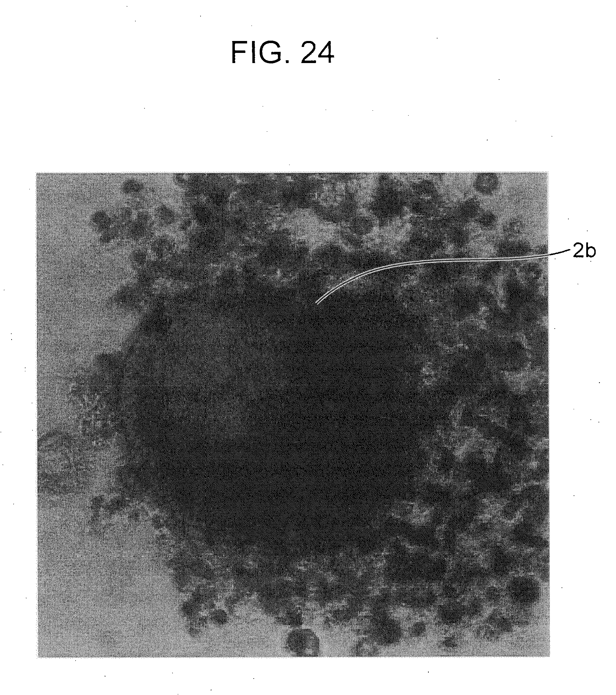

FIG. 24 is a microphotograph of a cell aggregate having an uneven density.

### DESCRIPTION OF EMBODIMENTS

### <Suction Tip>

### (First Embodiment)

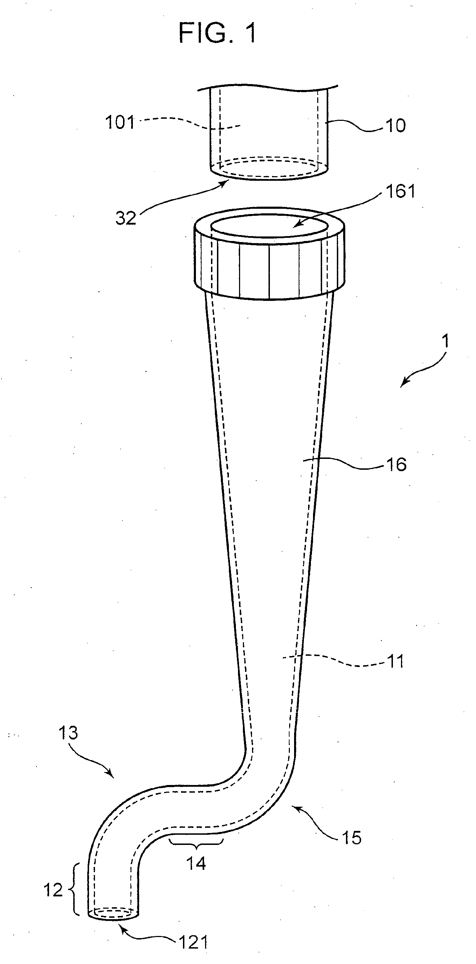

In the following, a suction tip 1 in the first embodiment of the invention is described in detail referring to the drawings. FIG. 1 is a schematic diagram illustrating a configuration of the suction tip 1 in the embodiment.

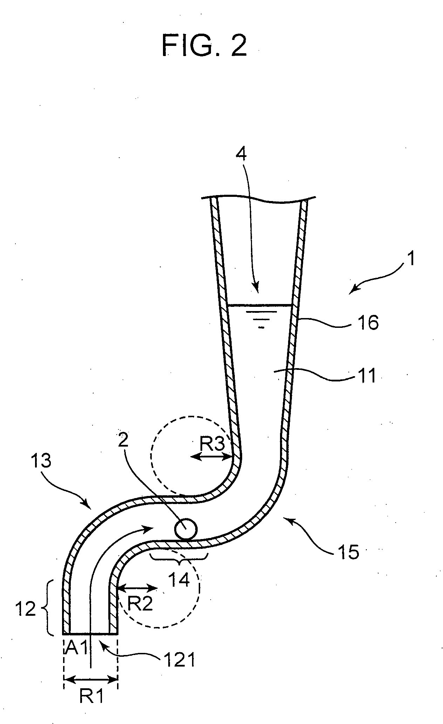

The suction tip 1 in the embodiment is a jig to be connected to a suction pipette 10 (suction means) for use in sucking a cell aggregate 2 (a spheroid or an object, see FIG. 2). As will be described later, the suction pipette 10 is a tubular member capable of generating a suction force. The suction pipette 10 is allowed to suck the cell aggregate 2 from a suction port 121, which is an opening formed in one end of a tubular member 11 of the suction tip 1 by generating a suction force in a tubular passage 101 in a state that the suction pipette 10 is connected to the suction tip 1.

The suction tip 1 is internally provided with a tubular passage 11 serving as a suction path for sucking the cell aggregate 2. The suction tip 1 is oriented in a substantially vertical direction when it is used. The suction tip 1 is provided with a distal end portion 12 including a suction port 121 for sucking the cell aggregate 2, the suction port 121 being an opening formed in one end of the tubular passage 11; a converting portion 13 continuously formed with the distal end portion 12, and configured to convert the extending direction of the tubular passage 11 in the distal end portion 12 in a horizontal direction; a horizontal portion 14 (trap portion) formed downstream of the converting portion 13 in the suction direction, and configured to trap the cell aggregate 2; a re-converting portion 15 formed downstream of the horizontal portion 14 in the suction direction, and configured to re-convert the extending direction of the tubular passage 11 in a substantially vertical direction; and a main body portion 16 formed downstream of the re-converting portion 15 in the suction direction and including a connection port 161 to be connected to the suction pipette 10, the connection port 161 being an opening formed in the other end of the tubular passage 11.

The size (inner volume) of the suction tip 1 is not specifically limited. The suction tip 1 may have an inner volume in the range of from 1 µL to 5 mL according to the purpose of use. In the embodiment, the inner volume of the suction tip 1 is 20 µL.

The material constituting the suction tip 1 is not specifically limited. Any material used for an ordinary suction tip 1 may be used. Examples of the material of the suction tip 1 are resin such as polypropylene or polystyrene, or glass. Use of resin as a material of the suction tip 1 allows for the suction tip 1 to appropriately expand the diameter of the connection port 161 when the connection port 161 formed in the main body portion 16 to be described later is connected to the suction pipette 10. The suction tip 1 is connected to the suction pipette 10 while securely coming into firm contact with the suction pipette 10. In the embodiment, the suction tip 1 is made of polypropylene.

The thickness of the tubular wall of the suction tip 1 is not specifically limited. An exceedingly small thickness, however, may lower the strength. In view of the above, the thickness of the tubular wall of the suction tip 1 is preferably in the range of from about 50 to 600 µm. In the embodiment, the thickness of the suction tip 1 is about 100 µm.

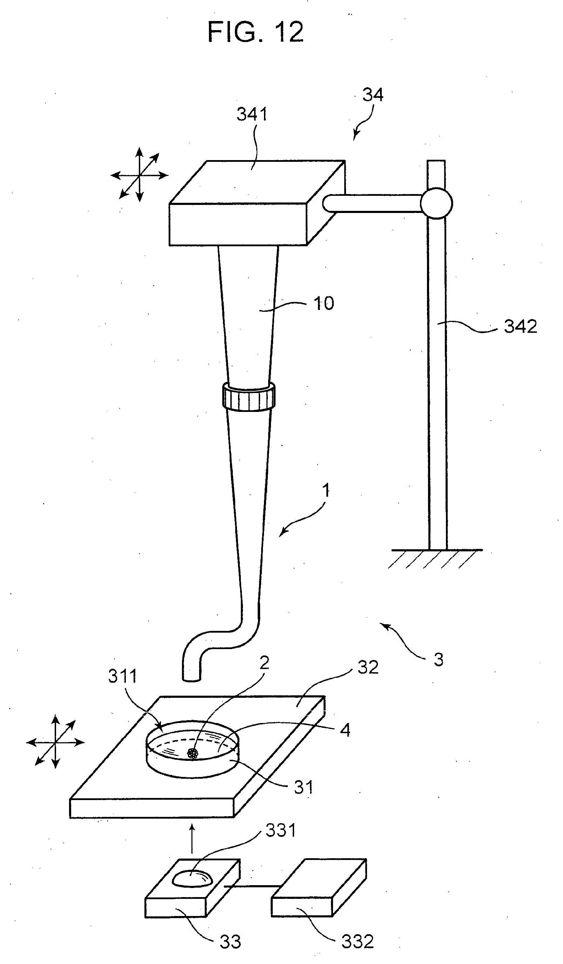

The cell aggregate 2 (object) is a sample to be sucked using the suction tip 1 in the embodiment. The cell aggregate 2 is normally held in a state that the cell aggregate 2 is contained in a liquid such as PBS (phosphate buffered saline), a cell culture solution, or a cell treatment solution in order to prevent deterioration due to drying or the like. In the embodiment, a liquid 4 is stored in a Petri dish 31 (vessel) constituted of a bottomed hollow cylindrical body having an inner bottom portion 311 and an upper end opened upward. The cell aggregate 2 is contained in the liquid 4 (see FIG. 12).

In sucking the cell aggregate 2 or in allowing the user to observe the cell aggregate 2 trapped in the horizontal portion 14 (trap portion) of the suction tip 1 by an object observation device 3 to be described later, it is preferable to remove impurities such as cell organelles, or cells that do not form an aggregate in advance from the liquid 4. The method for removing these matters is not specifically limited. It is possible to use a method of passing a cell culture solution containing the cell aggregate 2 and impurities through a filter or the like for removal. Passing the solution through a filter makes it possible to obtain the liquid 4 such as a cell culture solution mainly containing the cell aggregate 2 of a specific size and impurities of a substantially same size.

Cells derived from a living body such as the cell aggregate 2 have a relatively large deviation in the shape, and the sizes of the cells are very small. Further, it is necessary to accurately operate the suction tip 1 having the suction port 121 of a very small size in order to suck only the small cell aggregate 2 to be sucked in the co-existence of impurities. The suction tip 1 in the embodiment is configured such that the suction port 121 is formed in the distal end portion 12 of the main body portion 16. Therefore, it is easy for the user to accurately move the suction port 121 close to the cell aggregate 2. Further, it is possible to hold the sucked cell aggregate 2 in the horizontal portion 14 (trap portion) to be described later. Therefore, unlike a case of using a conventional suction tip (see FIG. 22C), the cell aggregate 2 is not discharged through the suction port 121 even in a state that the suction port 121 is immersed in the liquid 4. Thus, the user is not required to take out the suction port 121 to such a height that the user can check whether the suction operation is successful. Further, the sucked cell aggregate 2 is trapped in the horizontal portion 14. Therefore, the user can easily check the shape or the like of the cell aggregate 2 by observing the horizontal portion 14 by an imaging device 33 (observing means) provided in the object observation device 3 to be described later. This is advantageous in contributing to enhanced work efficiency in the bio-related technology or in the pharmaceutical field. In particular, at the time of observation by the imaging device 33, the suction port 121 is immersed in the liquid 4, and it is not necessary for the user to take out the suction port 121 from the liquid 4 so that the cell aggregate 2 does not fall. Therefore, the distance between the cell aggregate 2 trapped in the horizontal portion 14 and an optical lens system of the imaging device 33 is shorter than the conventional configuration. This makes it easy for the user to capture an image of the cell aggregate 2 held in the horizontal portion 14 within the depth of field of the optical lens system.

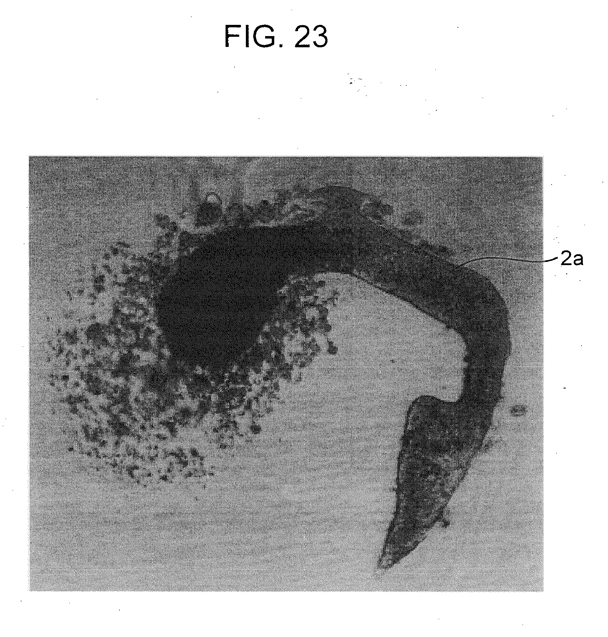

Further, use of the cell aggregate 2 is advantageous, as compared with a test result obtained by using one cell, in that an environment similar to the environment in the living body is re-configured within the cell aggregate 2, taking into consideration the interaction between the cells. It is possible to obtain a result taking into consideration the functions of the individual cells, and to adjust an experiment condition to be in conformity with a condition suitable for the environment in the living body. In view of the above, use of the cell aggregate 2 is important in the regenerative medical field and in the field of developing pharmaceuticals such as anticancer agents. Practical examples of the cell aggregate 2 are BxPC-3 (human pancreatic adenocarcinoma cells), embryonic stem cells (ES cells), and induced pluripotent stem cells (iPS cells). Generally, such a cell aggregate 2 is formed by flocculation of several to several hundred thousands of cells. Therefore, the sizes of the cell aggregates 2 differ from each other. A cell aggregate 2 formed of living cells has a substantially spherical shape. However, when a part of the cells constituting a cell aggregate 2 is modified or dead, a cell aggregate 2a may have a distorted shape as illustrated in FIG. 23, or a cell aggregate 2b may have an uneven density as illustrated in FIG. 23. The suction tip 1 in the embodiment is configured such that the cell aggregate 2 can be trapped in the trap portion 14, and the shape of the cell aggregate 2 can be easily observed from the outside. Thus, it is possible to obtain a result having enhanced reliability in the bio-related technology or in the pharmaceutical field (including the regenerative medical field and in the field of developing pharmaceuticals such as anticancer agents) as described above.

The shape of the Petri dish 31 is not specifically limited. Preferably, in view of operability and stability, the Petri dish 31 has a flat shape, and is provided with the inner bottom portion 311 of a flat surface, with the height of the Petri dish 31 being relatively smaller than the lateral width. The opening width and the depth (height) of the Petri dish 31 are not specifically limited. As far as it is possible to immerse the distal end portion 12 including the suction port 121 of the suction tip 1, the Petri dish 31 may have any size.

At least a part of the Petri dish 31 is made of a light transmissive material so that the user can observe the cell aggregate 2 sunk on the inner bottom portion 311 of the Petri dish 31 from below by the imaging device 33 included in the object observation device 3 to be described later.

The light transmissive material is not specifically limited. For instance, it is preferable to use a thermoplastic resin, a thermoset resin, or a photocured resin. More specifically, examples of the light transmissive material include polyethylene resin; polyethylene naphthalate resin; polypropyrene resin; polyimide resin: polyvinyl chloride resin; cycloolefin copolymer; norbornene containing resin; polyether sulfonic resin; polyethylene naphthalate resin; cellophane; aromatic polyamide resin; (meth)acrylic resin such as polymethylmethacrylate; styrene resin such as polystyrene, styrene-acrylonitrile copolymer; polycarbonate resin; polyester resin; phenoxy resin; butyral resin; polyvinyl alcohol; cellulose resin such as ethyl cellulose, cellulose acetate, and cellulose acetate butylate; epoxy resin; phenol resin; silicone resin; and polylactic acid. Further, it is preferable to use inorganic materials, for instance, metal alkoxide, ceramic precursor polymer, or a solution obtained by subjecting a solution containing metal alkoxide to hydrolytic polymerization by a sol-gel process, or inorganic materials obtained by solidifying a combination of these compounds, for instance, an inorganic material having siloxane bond (such as polydimethyl siloxane) or glass. Examples of glass are soda glass, quartz, borosilicate glass, Pyrex (registered trademark) glass, low melting-point glass, photosensitive glass, and a variety of optical glass materials having a variety of refractive indexes and Abbe numbers.

As an example that satisfies these requirements, there is proposed a cylindrical Petri dish 31 made of glass and having a height of about several millimeters to several centimeters, and a diameter of about 10 cm. In the embodiment, there is used a cylindrical Petri dish 31 made of glass with a height of 15 mm and a diameter of 60 mm.

The liquid 4 such as a cell culture solution to be stored in the Petri dish 31 is not specifically limited. Any liquid 4 can be stored, as far as the liquid 4 does not deteriorate the properties of the cell aggregate 2. Examples of the liquid 4 are media such as a basal medium, a synthetic medium, an Eagle's medium, an RPMI medium, a Fischer's medium, a Ham's medium, a MCDB medium, and a serum; and glycerol to be added before cryopreservation, a cell cryopreserved solution provided by a cell banker (Juji Field Inc.), formalin, a reagent for fluorescent dyeing, an antibody, purified water, and saline. More specifically, when the cell aggregate 2 is BxPC-3 (human pancreatic adenocarcinoma cells), which are cells derived from a living body, the liquid 4 may be a solution obtained by containing 10% FBS (Fetal Bovine Serum) in an RPMI-1640 medium, with addition of supplements such as an antibiotic or pyruvate sodium, as necessary.

Returning to the description on the suction tip 1, the suction port 121 is an opening formed in one end of the tubular passage 11, and is formed in the distal end portion 12. The diameter of the suction port 121 is not specifically limited. As far as it is possible to suck the cell aggregate 2, the diameter of the suction port 121 may have any size. The diameter of the suction port 121 is set to be smaller than the diameter of the connection port 161, which is an opening formed in the other end of the tubular passage 11. According to this configuration, the suction tip 1 can generate a large suction force around the suction port 121 during a suction operation. In the embodiment, the diameter of the suction port 121 is 300 µm.

The distal end portion 12 is a portion disposed in a substantially vertical direction when in use, and configured such that a part of the distal end portion 12 including the suction port 121 is immersed in the liquid 4 when the cell aggregate 2 is sucked. The user disposes the suction port 121 at a position above the cell aggregate 2 sunk in the liquid 4 stored in the Petri dish 31, and moves the distal end portion 12 downward from the aforementioned position. This allows for the user to move the suction port 121 sufficiently close to the cell aggregate 2 in a state that a part of the distal end portion 12 including the suction port 121 is immersed in the liquid 4, while preventing generation of a flow of the liquid 4 around the cell aggregate 2. The method for moving the suction port 121 close to the cell aggregate 2 is not specifically limited. The suction port 121 may be moved down in a substantially vertical direction so that the suction port 121 comes close to the cell aggregate 2, or the suction port 121 may be moved obliquely downward so that the suction portion 121 comes close to the cell aggregate 2.

The length of the distal end portion 12 is not specifically limited. As far as it is possible to move the suction port 121 to such a position that the cell aggregate 2 sunk in the liquid 4 stored in the Petri dish 31 can be sucked, the length of the distal end portion 12 may be optionally set. The length of the distal end portion 12 may be determined, as necessary, depending on the distance from the liquid surface of the stored liquid 4 to the inner bottom portion 311 of the Petri dish 31, or depending on the size of the cell aggregate 2. In the embodiment, the length of the distal end portion 12 is set to 1 mm.

The distal end portion 12 includes the tubular passage 11 extending in a direction substantially orthogonal to the radial direction of the suction port 121 (in other words, in a substantially vertical direction when in use). The tubular passage 11 in the distal end portion 12 is a linear conduit. The inner diameter of the tubular passage 11 is set to 500 µm. The cell aggregate 2 sucked through the suction port 121 travels downstream in the suction direction through the tubular passage 11, and reaches the converting portion 13.

The converting portion 13 is a portion continuously formed with the distal end portion 12, and configured to convert the extending direction of the tubular passage 11 in the distal end portion 12 in a horizontal direction. The shape of the converting portion 13 is not specifically limited. The converting portion 13 may be formed by bending the tubular passage 11 extending in the substantially vertical direction in the distal end portion 12 substantially at a right angle to extend in a horizontal direction; or may be formed by gradually bending the tubular passage 11 in a horizontal direction. In the embodiment, the inner diameter of the tubular passage 11 in the converting portion 13 is the same as the inner diameter of the tubular passage in the distal end portion 12. The tubular passage 11 in the converting portion 13 is curved into such a shape that the curved surface of the tubular passage 11 comes into contact with the circumference of a 1/4 circle having a radius R2, which is the same as a diameter R1 of a section of the distal end portion 12 of the suction tip 1 (see FIG. 2). The cell aggregate 2 travels further downstream in the suction direction via the converting portion 13, and reaches the horizontal portion 14.

The horizontal portion 14 (trap portion) is a portion formed downstream of the converting portion 13 in the suction direction, and configured to trap the cell aggregate 2 sucked through the suction port 121 in a horizontal state. In the horizontal portion 14, the cell aggregate 2 sinks without traveling to the main body portion 16 to be described later; or travels downstream in the suction direction via the horizontal portion 14 to the main body portion 16, and then travels upstream in the suction direction while sinking in the gravitational direction, and returns to the horizontal portion 14 for trapping the cell aggregate 2.

More specifically, during a suction operation, the cell aggregate 2 flows along with a flow of the liquid 4 being sucked, and travels downstream in the suction direction. However, when the suction operation is completed or the suction force is weakened, the flow of liquid disappears or is weakened. Then, the cell aggregate 2 starts to sink in the gravitational direction. FIG. 2 is a schematic diagram illustrating a behavior of the cell aggregate 2 to be sucked using the suction tip 1 in the embodiment. As illustrated in FIG. 2, when a cell aggregate 2 exists in the tubular passage 11 in the horizontal portion 14 at a point of time when sinking in the gravitation direction is started, the cell aggregate 2 sinks in the horizontal portion 14 and is trapped. In FIG. 2, the arrow A1 indicates a behavior of the cell aggregate 2 that is sucked through the suction port 121, sinks and is trapped in the horizontal portion 14.

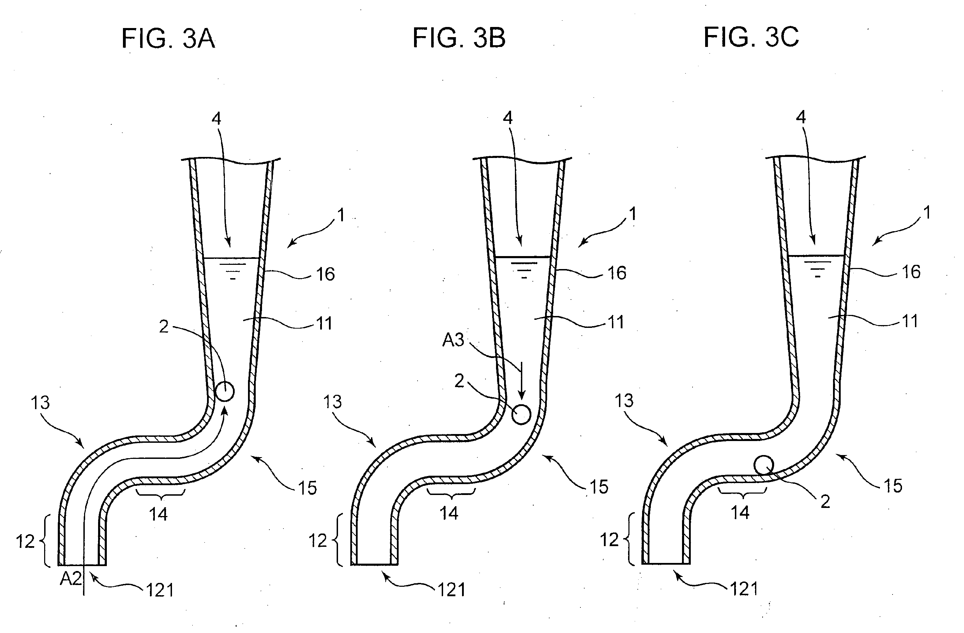

FIGS. 3A to 3C are schematic diagrams illustrating a behavior of the cell aggregate 2 to be sucked using the suction tip 1 in the embodiment. As illustrated in FIG. 3A, when the cell aggregate 2 travels to the tubular passage 11 in the main body portion 16 by the point of time when sinking in the gravitational direction is started, as illustrated in FIG. 3B, the cell aggregate 2 travels upstream in the suction direction accompanied by sinking in the gravitational direction, and as illustrated in FIG. 3C, the cell aggregate 2 returns to the horizontal portion 14 and is trapped in the horizontal portion 14. The arrow A2 in FIG. 3A indicates a behavior of the cell aggregate 2 that is sucked through the suction port 121 and travels to the main body portion 16. The arrow A3 in FIG. 3B indicates a behavior of the cell aggregate 2 that falls upstream in the suction direction from the main body portion 16 in the gravitational direction.

In any case, the horizontal portion 14 is disposed substantially horizontally when in use. Therefore, the cell aggregate 2 trapped in the horizontal portion 14 in a state that a flow of liquid disappears is held in a stationary state, without being moved from the sinking position.

The horizontal length of the horizontal portion 14 is not specifically limited. The length of the horizontal portion 14 may be set to zero or larger, for instance. A case, in which the length of the horizontal portion 14 is zero indicates a case, in which the downstream end of the converting potion 13 in the suction direction is continuously formed with the upstream end of the re-converting portion 15 to be described later in the suction direction. In this case, a horizontal part is formed in the tubular passage 11 only at a connecting portion between the converting portion 13 and the re-converting portion 15. In the embodiment, the horizontal part is called as the horizontal portion 14 whose length is zero. Further, the upper limit of the length of the horizontal portion 14 is set to a length required for the cell aggregate 2 traveling upstream in the suction direction to prevent from reaching the converting portion 13 or the distal end portion 12 via the horizontal portion 14, when the cell aggregate 2 that has traveled to the main body portion 16 sinks in the gravitational direction and returns to the horizontal portion 14, taking into consideration the quantity of the liquid 4 to be sucked, the inner volume of the suction tip 1, the resistive force of the cell aggregate 2 against the liquid 4, the weight of the cell aggregate 2, the position (height) of the cell aggregate 2 when the cell aggregate 2 starts to sink in the gravitational direction, the viscosity or the specific gravity of the liquid 4 to be sucked together with the cell aggregate 2, the amount of impurities, and the frictional coefficient of the inner wall of the tubular passage 11. In the embodiment, the length of the horizontal portion 14 is set to 1 mm.

In the embodiment, a "horizontal" degree of the horizontal portion 14 is a degree at which the cell aggregate 2 can be kept in a stationary state without moving by the weight thereof. Specifically, even if the portion where the cell aggregate 2 is held is not in a horizontal state in a strict sense and is inclined when in use, as far as the tilt of the portion is such that the cell aggregate 2 can be kept in a stationary state without moving by the weight thereof, the portion is included in the horizontal portion 14 in the embodiment.

The shape of the cell aggregate 2 to be trapped in the horizontal portion 14 can be easily checked by the imaging device 33 provided in the object observation device 3 to be described later. Further, the cell aggregate 2 trapped in the horizontal portion 14 is simply held stationary in a state that the cell aggregate 2 sinks. Therefore, once a flow of liquid flowing upstream in the suction direction is generated at the time of ejection, the cell aggregate 2 is easily moved upstream in the suction direction together with the flow of liquid, and is ejected.

The re-converting portion 15 is a portion formed downstream of the horizontal portion 14 in the suction direction, and configured to convert the extending direction of the tubular passage 11 in a substantially vertical direction (upward with respect to a horizontal direction). The shape of the re-converting portion 15 is not specifically limited. The shape of the re-converting portion 15 may be formed by bending the tubular passage 11 extending in the horizontal direction in the horizontal portion 14 substantially at a right angle to extend in a substantially vertical direction; or may be formed by gradually bending the tubular passage 11 in a substantially vertical direction. In the embodiment, the inner diameter of the tubular passage in the re-converting portion 15 is the same as the inner diameter of the tubular passage 11 in the distal end portion 12. The tubular passage 11 in the re-converting portion 15 is curved into such a shape that the curved surface of the tubular passage 11 comes into contact with the circumference of a 1/4 circle having a radius R3, which is the same as the diameter R1 of the section of the distal end portion 12 of the suction tip 1 (see FIG. 2). The cell aggregate 2 travels further downstream in the suction direction via the re-converting portion 15, and reaches the main body portion 16; or the cell aggregate 2 sinks in the gravitational direction as the suction operation is completed or the suction force is weakened in the re-converting portion 15, travels upstream in the suction direction, and reaches the horizontal portion 14.

The main body portion 16 is a portion formed downstream of the re-converting portion 15 in the suction direction. The main body portion 16 is disposed in a substantially vertical direction when in use, and includes the tubular passage 11 whose inner diameter increases toward downstream in the suction direction. The opening formed in the downstream end of the tubular passage 11 in the suction direction functions as the connection port 161 to be connected to the suction pipette 10 (suction means). The diameter of the connection port 161 is not specifically limited. The diameter of the connection port 161 is determined, as necessary, taking into consideration the diameter of a connected port 32 (see FIG. 1) of the suction pipette 10 to be used. In the embodiment, the diameter of the connection port 161 is set to 3,000 µm. The suction tip 1 in the embodiment is configured such that forming the connection port 161 to have a relatively large size makes it possible to enhance the strength of the main body portion 16. This is advantageous in securely connecting the suction tip 1 to the suction pipette 10.

When the cell aggregate 2 reaches the main body portion 16, the cell aggregate 2 starts to sink in the gravitation direction within the tubular passage 11 at a point of time when the suction operation is completed or the suction force is weakened. As described above, the tubular passage 11 has such a shape that the inner diameter thereof increases toward downstream in the suction direction (in other words, the inner diameter decreases toward upstream in the suction direction). Therefore, the inner wall of the tubular passage 11 functions as a tapered surface capable of correcting the sinking direction so that the sinking cell aggregate 2 reaches the re-converting portion 15. The cell aggregate 2 reaches the horizontal portion 14 via the re-converting portion 15, and is trapped in the horizontal portion 14.

As described above, the suction tip 1 in the embodiment is configured such that the suction port 121 is formed in one end of the distal end portion 12, which is disposed in a substantially vertical direction when in use. Therefore, it is possible to suck the cell aggregate 2 accurately in a state that the suction port 121 comes sufficiently close to the cell aggregate 2, which is held in the liquid 4 stored in the Petri dish 31, for instance. The sucked cell aggregate 2 is trapped in a stationary state in the horizontal portion 14. Therefore, the cell aggregate 2 is not discharged through the suction port 121, even when the suction port 121 is immersed in the liquid 4. Further, the cell aggregate 2 trapped in the horizontal portion 14 is easily ejected together with a flow of liquid when the flow of liquid flowing upstream in the suction direction is generated at the time of ejection. Further, when the user observes the trapped cell aggregate 2 by the object observation device 3 to be described later, the user can easily check the shape of the cell aggregate 2 simply by observing the horizontal portion 14.

### (Second Embodiment)

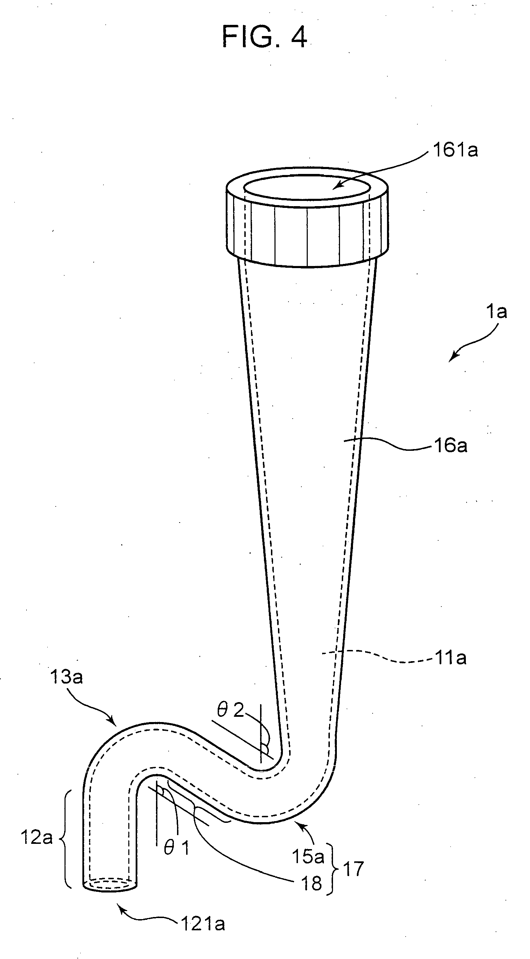

In the following, a suction tip 1a in the second embodiment of the invention is described in detail referring to the drawings. FIG. 4 is a schematic diagram illustrating a configuration of the suction tip 1a in the embodiment. The suction tip 1a in the embodiment is internally provided with a tubular passage 11a serving as a suction path for sucking a cell aggregate 2. The suction tip 1a is provided with a distal end portion 12a disposed in a substantially vertical direction when in use and including a suction port 121a for sucking the cell aggregate 2, the suction port 121 a being an opening formed in one end of the tubular passage 11a; a converting portion 13a continuously formed with the distal end portion 12a, and configured to convert the extending direction of the tubular passage 11a in the distal end portion 12a downward with respect to a horizontal direction; a trap portion 17 formed downstream of the converting portion 13a in the suction direction, and configured to trap the cell aggregate 2 to be sucked through the suction port 121a; and a main body portion 16a formed downstream of the trap portion 17 in the suction direction and including a connection port 161 a to be connected to a suction pipette 10, the connection port 161 a being an opening formed in the other end of the tubular passage 11a. The trap portion 17 includes a re-converting portion 15a configured to re-convert the extending direction of the tubular passage 11 a in a substantially vertical direction (upward with respect to a horizontal direction), and to trap the cell aggregate 2.

The distal end portion 12a has substantially the same configuration as the distal end portion 12 described in detail in the first embodiment, and therefore, description of the distal end portion 12a is omitted.

The converting portion 13a is a portion continuously formed with the distal end portion 12a, and configured to convert the extending direction of the tubular passage 11 a in the distal end portion 12a downward with respect to a horizontal direction. The shape of the converting portion 13a is not specifically limited. The converting portion 13a may be formed by bending the tubular passage 11 a extending in a substantially vertical direction in the distal end portion 12a into a V-shape to extend downward; or may be formed by bending the tubular passage 11a into a U-shape to extend downward. The angle θ1 defined by the tubular passage 11a in the distal end portion 12a, and the tubular portion 11 a in the converting portion 13a where the extending direction is converted is not specifically limited. The angle θ1 may be an angle exceeding 90° but not larger than 180°. In the embodiment, the angle θ1 is set to 120°.

The converting portion 13a has a folded shape bulging upward. According to this configuration, the cell aggregate 2 traveling downstream in the suction direction via the converting portion 13a cannot travel upstream in the suction direction via the converting portion 13a simply by falling in the gravitational direction after the suction operation is completed or the suction force is weakened. The cell aggregate 2 travels further downstream in the suction direction via the converting portion 13a, and reaches the trap portion 17.

The trap portion 17 is a portion formed downstream of the converting portion 13a in the suction direction. The trap portion 17 includes a re-converting portion 15a for re-converting the extending direction of the tubular passage 11a in the converting portion 13a whose extending direction is converted to extend in a substantially vertical direction, and a connecting portion 18 for connecting between the converting portion 13a and the re-converting portion 15a. The cell aggregate 2 is trapped in the re-converting portion 15a.

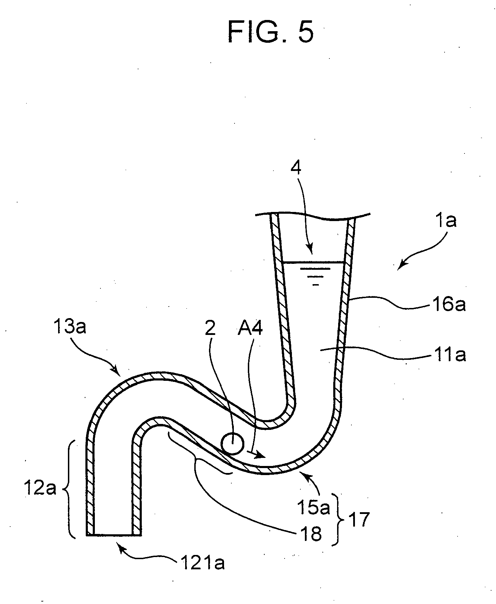

The connecting portion 18 is a portion for connecting the downstream end of the converting portion 13a in the suction direction, and the upstream end of the re-converting portion 15a in the suction direction. The connecting portion 18 is provided with the linearly extending tubular passage 11 a. The connecting portion 18 is disposed to incline downward from upstream toward downstream in the suction direction. FIG. 5 is a schematic diagram illustrating a behavior of the cell aggregate 2 to be sucked by using the suction tip 1a in the embodiment. As illustrated in FIG. 5, even when the cell aggregate 2 has not reached the re-converting portion 15a to be described later or to the main body portion 16a by the point of time when the suction operation is completed or the suction force is weakened, the cell aggregate 2 sunk in the connecting portion 18 in the gravitational direction travels downstream in the suction direction, while rolling on the inner wall of the tubular passage 11a of the connecting portion 18, reaches the re-converting portion 15a, and is trapped in the re-converting portion 15a. In FIG. 5, the arrow A4 indicates a behavior of the cell aggregate 2 travelling downstream in the suction direction in the tubular passage 11a in the connecting portion 18.

The length of the connecting portion 18 is not specifically limited. However, it is necessary to move the suction port 121 a sufficiently close to the cell aggregate 2 when the suction port 121a formed in the distal end portion 12a is immersed in a liquid 4 holding the cell aggregate 2 for sucking the cell aggregate 2. In view of the above, the length of the connecting portion 18 is adjusted to such a length that the height position of the re-converting portion 15a to be described later is higher than the height position of the suction port 121a when the suction tip 1a is used. Further, the connecting portion 18 itself may be omitted, and the downstream end of the converting portion 13a in the suction direction may be directly connected to the upstream end of the re-converting portion 15a in the suction direction.

The re-converting portion 15a is a portion formed downstream of the connecting portion 18 in the suction direction, and configured to re-convert the extending direction of the tubular passage 11a in a substantially vertical direction (upward with respect to a horizontal direction). The shape of the re-converting portion 15a is not specifically limited. The re-converting portion 15a may be formed by bending the tubular passage 11a in the converting portion 13a, whose extending direction is bent downward by the angle θ1, by the angle θ2 to extend in a substantially vertical direction; or may be formed by gradually bending the tubular passage 11 a to extend in a substantially vertical direction. In the embodiment, the inner diameter of the tubular passage 11 a in the re-converting portion 15a is the same as the inner diameter of the tubular passage in the distal end portion 12a. The tubular passage 11a is a tubular passage obtained by bending the tubular passage 11a in the converting portion 13a, whose extending direction is bent downward by 120°, by 120° to extend in a substantially vertical direction. The cell aggregate 2 whose traveling direction is converted by the re-converting portion 15a travels further downstream in the suction direction, and reaches the main body portion 16a.

The main body portion 16a has substantially the same configuration as the main body portion 16 described in detail in the first embodiment, and therefore, repeated description thereof is omitted.

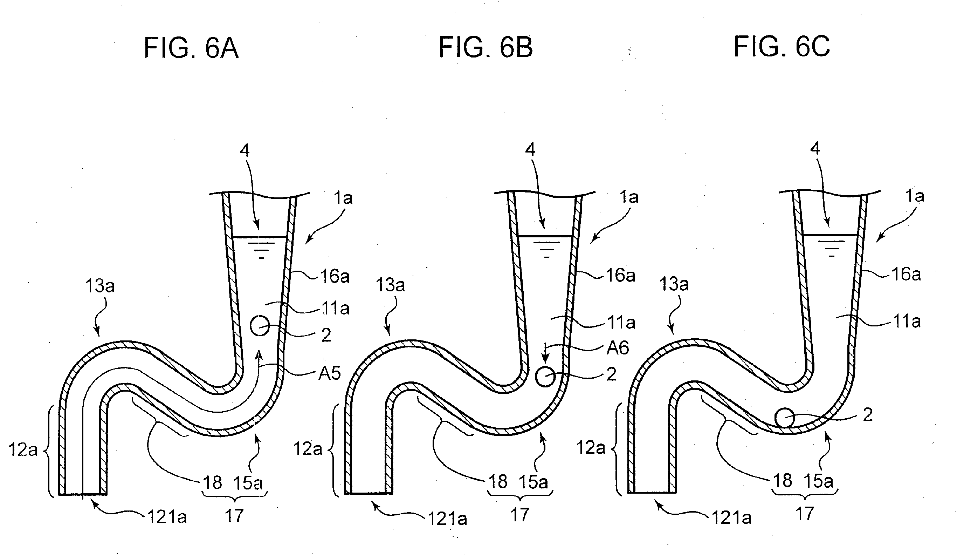

FIGS. 6A to 6C are schematic diagrams illustrating a behavior of the cell aggregate 2 to be sucked by using the suction tip 1a in the embodiment. As illustrated in FIG. 6A, when the cell aggregate 2 reaches the main body portion 16a, as illustrated in FIG. 6B, the cell aggregate 2 sinks in the gravitational direction within the tubular passage 11 a at a point of time when the suction operation is completed or the suction force is weakened. The downstream portion of the re-converting portion 15a in the suction direction and the upstream portion of the main body portion 16a in the suction direction are continuously formed. Therefore, as illustrated in FIG. 6C, the cell aggregate 2 sinking in the tubular passage 11a in the main body portion 16a reaches the re-converting portion 15a, and is trapped in the re-converting portion 15a. In FIG. 6A, the arrow A5 indicates a behavior of the cell aggregate 2 that is sucked through the suction port 121 a and travels to the main body portion 16a. In FIG. 6B, the arrow A6 indicates a behavior of the cell aggregate 2 that falls upstream in the suction direction from the main body portion 16a in the gravitational direction.

As described above, the suction tip 1a in the embodiment is configured such that the extending direction of the tubular passage 11a is converted downward by the converting portion 13a. When in use, the converting portion 13a has a folded shape bulging upward. According to this configuration, an object that has been sucked via the converting portion 13a cannot cross over the converting portion 13a simply by falling in the gravitational direction. Thus, the object is not ejected through the suction port 121a. Further, both of a cell aggregate 2 that has reached the main body portion 16a, and a cell aggregate 2 present within the tubular passage 11 a in the connecting portion 18 by the point of time when the suction operation is completed or the suction force is weakened sink in the gravitational direction, and are trapped in the re-converting portion 15a. Therefore, when the user observes the trapped cell aggregate 2 by the object observation device 3 to be described later, the user is only required to observe the re-converting portion 15a. This eliminates the need of searching the cell aggregate 2, and allows for the user to easily check the shape of the trapped cell aggregate 2.

### (Third Embodiment)

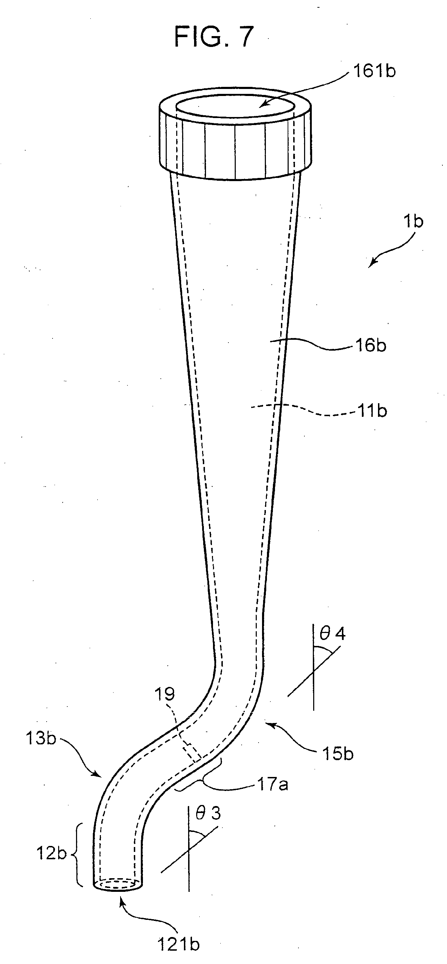

In the following, a suction tip 1b in the third embodiment of the invention is described in detail referring to the drawings. FIG. 7 is a schematic diagram illustrating a configuration of the suction tip 1b in the embodiment. The suction tip 1b in the embodiment is internally provided with a tubular passage 11b serving as a suction path for sucking a cell aggregate 2. The suction tip 1b is provided with a distal end portion 12b oriented in a substantially vertical direction when in use and including a suction port 121b for sucking a cell aggregate 2, the suction port 121 b being an opening formed in one end of the tubular passage 11b; a converting portion 13b continuously formed with the distal end portion 12b, and configured to convert the extending direction of the tubular passage 11b in a direction obliquely upward with respect to a horizontal direction; a trap portion 17a formed downstream of the converting portion 13b in the suction direction, and configured to trap the cell aggregate 2 to be sucked through the suction port 121b; a re-converting portion 15b formed downstream of the trap portion 17a in the suction direction, and configured to re-convert the extending direction of the tubular passage 11b in a substantially vertical direction; and a main body portion 16b formed downstream of the re-converting portion 15b in the suction direction and including a connection port 161b to be connected to a suction pipette 10, the connection port 161b being an opening formed in the other end of the tubular passage 11b. The trap portion 17a is provided with a projection piece 19 projecting from an inner wall of the tubular passage 11b.

The distal end portion 12b has substantially the same configuration as the distal end portion 12 described in detail in the first embodiment, and therefore, description thereof is omitted.

The converting portion 13b is a portion continuously formed with the distal end portion 12b, and configured to convert the extending direction of the tubular passage 11b obliquely upward with respect to a horizontal direction. The shape of the converting portion 13b is not specifically limited. The angle θ3 defined by the tubular passage 11b in the distal end portion 12b, and the tubular portion 11b in the converting portion 13b, where the extending direction is converted may be an angle exceeding zero but smaller than 90°. In the embodiment, the angle 03 is set to 45°. The cell aggregate 2 travels further downstream in the suction direction via the converting portion 13b, and reaches the trap portion 17a.

The trap portion 17a is a portion formed downstream of the converting portion 13b in the suction direction, and configured to trap the cell aggregate 2 to be sucked through the suction port 121 b. The trap portion 17a is provided with the linear tubular passage 11b. The tubular passage 11b is provided with a projection piece 19 projecting from the inner wall thereof. The trap portion 17a is disposed to incline upward from upstream toward downstream in the suction direction.

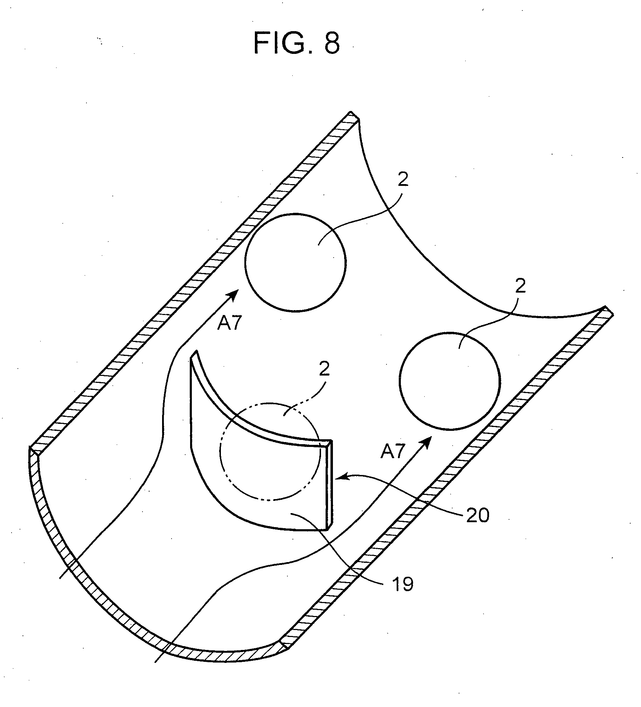

FIG. 8 is a schematic diagram illustrating a behavior of the cell aggregate 2 in the vicinity of the projection piece 19. The projection piece 19 is a plate-like member projecting from the inner wall of the tubular passage 11b to a central axis of the tubular passage 11b. An end of the projection piece 19 is joined to the lower portion of the inner wall of the tubular passage 11b in the trap portion 17a, and the other end thereof is a free end. Further, the projection piece 19 has a curved shape bulging upstream in the suction direction. According to this configuration, even when the cell aggregate 2 traveling downstream in the suction direction collides with the projection piece 19 during a suction operation, as illustrated by the arrow A7, the traveling direction of the cell aggregate 2 is corrected so that the cell aggregate 2 passes through the left and right spaces of the projection piece 19. The cell aggregate 2 travels further downstream in the suction direction while bypassing the projection piece 19. In FIG. 8, the cell aggregate 2 indicated by the solid line indicates the cell aggregate 2 traveling downstream in the suction direction during a suction operation.

The projection piece 19, and the lower portion of the inner wall of the tubular passage 11b joined to the projection piece 19 constitute a restraining portion 20. The restraining portion 20 is a portion for restraining traveling of the cell aggregate 2 that travels downstream in the suction direction while bypassing the projection piece 19, and thereafter sinks in the gravitational direction at a point of time when the suction operation is completed or the suction force is weakened for trapping the cell aggregate 2. In the restraining portion 20, the cell aggregate 2 is trapped in a state that the cell aggregate 2 comes into contact with the lower portion of the inner wall of the tubular passage 11b and with the projection piece 19, as indicated by the two-dotted chain line illustrated in FIG. 8.

The angle θ5 defined by the projection piece 19, and the tubular passage 11b connected to the projection piece 19 is not specifically limited. The angle θ5 is preferably be an angle exceeding zero but smaller than 180°. In order to restrain the cell aggregate 2 that sinks in the gravitational direction from the re-converting portion 15b or from the main body portion 16b and travels upstream in the suction direction from traveling, and to stably trap the cell aggregate 2, preferably, the angle θ5 is from 45° to 135°. In the embodiment, the angle θ5 is set to 90°.

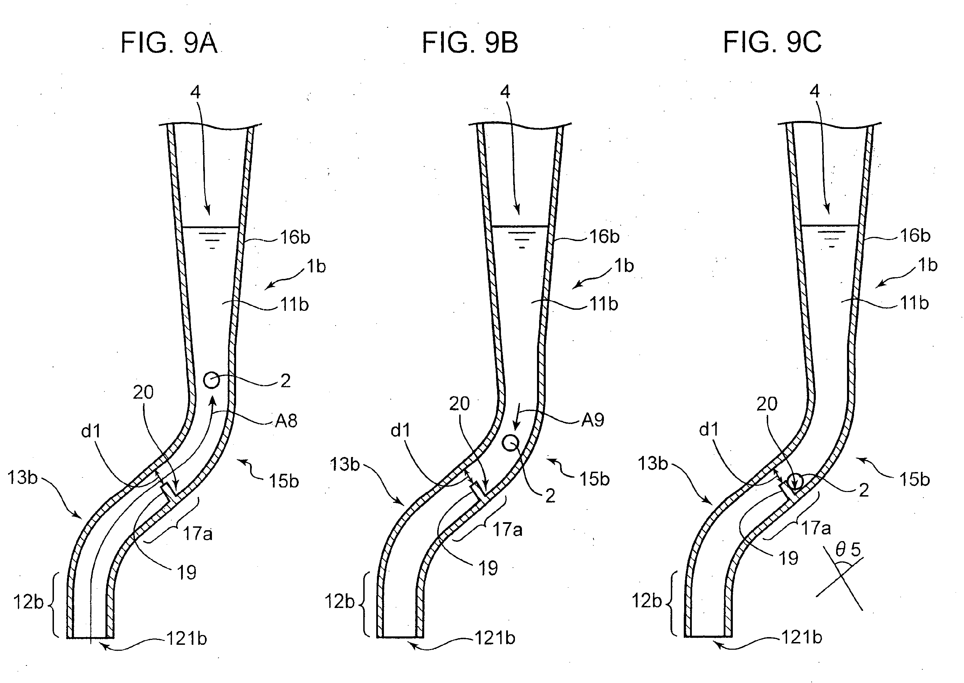

The length (height) of the projection piece 19 is not specifically limited. The length of the projection piece 19 is set, as necessary, taking into consideration the inner diameter of the tubular passage 11b or the diameter of the cell aggregate 2. In the embodiment, the projection piece 19 has the length 0.3 mm with respect to the inner diameter 0.6 mm of the tubular passage 11b. The distance d1 from the other end (free end) of the projection piece 19 to the upper portion of the inner wall of the tubular passage 11b is set to 0.3 mm (see FIG. 9A to FIG. 9C to be described later).

The cell aggregate 2 travels further downstream in the suction direction via the trap portion 17a, and reaches the re-converting portion 15b.

The re-converting portion 15b is a portion formed downstream of the trap portion 17a in the suction direction, and configured to convert the extending direction of the tubular passage 11b in a substantially vertical direction. The shape of the re-converting portion 15b is not specifically limited. The shape of the re-converting portion 15b may be a shape obtained by bending the tubular passage 11b in he converting portion 13b, whose extending direction is converted by the angle θ3 to extend obliquely upward, by the angle θ4 to extend in a substantially vertical direction. In the embodiment, the inner diameter of the tubular passage 11b in the re-converting portion 15b is the same as the inner diameter of the tubular passage in the distal end portion 12b. The tubular passage 11b is a tubular passage obtained by bending the tubular passage 11b in the converting portion 13b, whose extending direction is converted by 45° to extend obliquely upward, by 45° to extend in a substantially vertical direction. The cell aggregate 2 whose traveling direction is converted by the re-converting portion 15b travels further downstream in the suction direction, and reaches the main body portion 16b.

The main body portion 16b has substantially the same configuration as the main body portion 16 described in detail in the first embodiment, and therefore, repeated description thereof is omitted.

FIGS. 9A to 9C are schematic diagrams illustrating a behavior of the cell aggregate 2 to be sucked by using the suction tip 1 b in the embodiment. As illustrated in FIG. 9A, when the cell aggregate 2 reaches the main body portion 16b, as illustrated in FIG. 9B, at a point of time when the suction operation is completed or the suction force is weakened, the cell aggregate 2 sinks in the gravitational direction within the tubular passage 11b. The downstream portion of the re-converting portion 15b in the suction direction and the upstream portion of the main body portion 16b in the suction direction are continuously formed, and the upstream portion of the re-converting portion 15b in the suction direction and the downstream portion of the trap portion 17a in the suction direction are continuously formed. Further, the re-converting portion 15b is configured only to bend the traveling direction of the cell aggregate 2 sinking in the gravitational direction by the angle θ4. Therefore, the cell aggregate 2 sunk in the re-converting portion 15b continues to sink upstream in the suction direction, and reaches the restraining portion 20 of the trap portion 17a disposed in an inclined state. The projection piece 19 projects from the lower portion of the inner wall of the tubular passage 11b to the central axis of the tubular passage 11b. Therefore, as illustrated in FIG. 9C, the cell aggregate 2 cannot bypass the projection piece 19 simply by falling in the gravitational direction, and is trapped in the restraining portion 20. In FIG. 9A, the arrow A8 indicates a behavior of the cell aggregate 2 that is sucked through the suction port 121 b and travels to the main body portion 16b. In FIG. 9B, the arrow A9 indicates a behavior of the cell aggregate 2 falling upstream in the suction direction from the main body portion 16b in the gravitational direction.

As described above, the suction tip 1b in the embodiment is configured such that the cell aggregate 2 travels downstream in the suction direction while bypassing the projection piece 19, and thereafter, the cell aggregate 2 which tries to travel upstream in the suction direction is blocked by the projection piece 19, and is retrained from traveling upstream in the suction direction in the restraining portion 20 for trapping the cell aggregate 2. Further, the projection piece 19 projects from the lower portion of the inner wall of the tubular passage 11b to the central axis of the tubular passage 11b. Therefore, the object sucked downstream in the suction direction with respect to the position where the projection piece 19 is formed cannot cross over the projection piece 19 simply by falling in the gravitational direction. Thus, the cell aggregate 2 is trapped in the restraining portion 20, and is not ejected through the suction port 121b. Further, when the trapped cell aggregate 2 is observed by the object observation device 3 to be described later, the user is only required to observe the restraining portion 20. This eliminates the need of searching the cell aggregate 2, and allows for the user to easily check the shape of the trapped cell aggregate 2.

### (Fourth Embodiment)

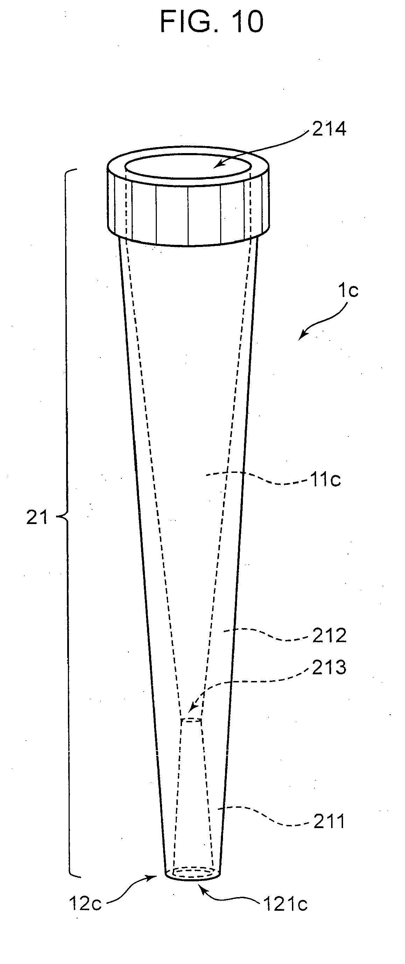

In the following, a suction tip 1c in the fourth embodiment of the invention is described in detail referring to the drawings. FIG. 10 is a schematic diagram illustrating a configuration of the suction tip 1c in the embodiment. The suction tip 1c in the embodiment is internally provided with a tubular passage 11c serving as a suction path for sucking a cell aggregate 2. The suction tip 1c is provided with a distal end portion 12c oriented in a substantially vertical direction when in use and including a suction port 121c for sucking the cell aggregate 2, the suction port 121 c being an opening formed in one end of the tubular passage 11c; and a reduced diameter portion 21 (trap portion) continuously formed with the distal end portion 12c, and including a connection port connected to a suction pipette 10, the connection port being an opening formed in the other end of the tubular passage 11c for trapping the cell aggregate 2.

The distal end portion 12c is oriented in a substantially vertical direction when in use, and includes the suction port 121c for sucking the cell aggregate 2. The suction port 121c is an opening formed in one end of the tubular passage 11c. The suction tip 1c in the embodiment includes the reduced diameter portion 21 having a tapered portion 211 configured such that the suction port 121c serves as an opening formed in one end of the tapered portion 211, and a through-hole 213 to be described later serves as an opening formed in the other end of the tapered portion 211. Specifically, the suction port 121c is a portion common to the distal end portion 12c and the reduced diameter portion 21. Therefore, unlike the distal end portion 12 in the first embodiment, the distal end portion 12c is not provided with a tubular passage of a predetermined length. In the suction tip 1c in the embodiment, a portion where the suction port 121c is formed is called as the distal end portion 12c.

The reduced diameter portion 21 is formed downstream of the distal end portion 12c in the suction direction. The reduced diameter portion 21 includes the tapered portion 211 for narrowing the flow channel of the tubular passage 11c toward downstream in the suction direction, and an inverse tapered portion 212 continuing from the tapered portion 211, and configured to expand the flow channel of the tubular passage 11c toward downstream in the suction direction.

The upstream end of the tapered portion 211 in the suction direction is joined to the inner wall of the tubular passage 11c, and the downstream end of the tapered portion 211 in the suction direction has the through-hole 213. During a suction operation, the cell aggregate 2 travels downstream in the suction direction along a tapered surface formed on the inner wall of the tapered portion 211, and reaches the through-hole 213.

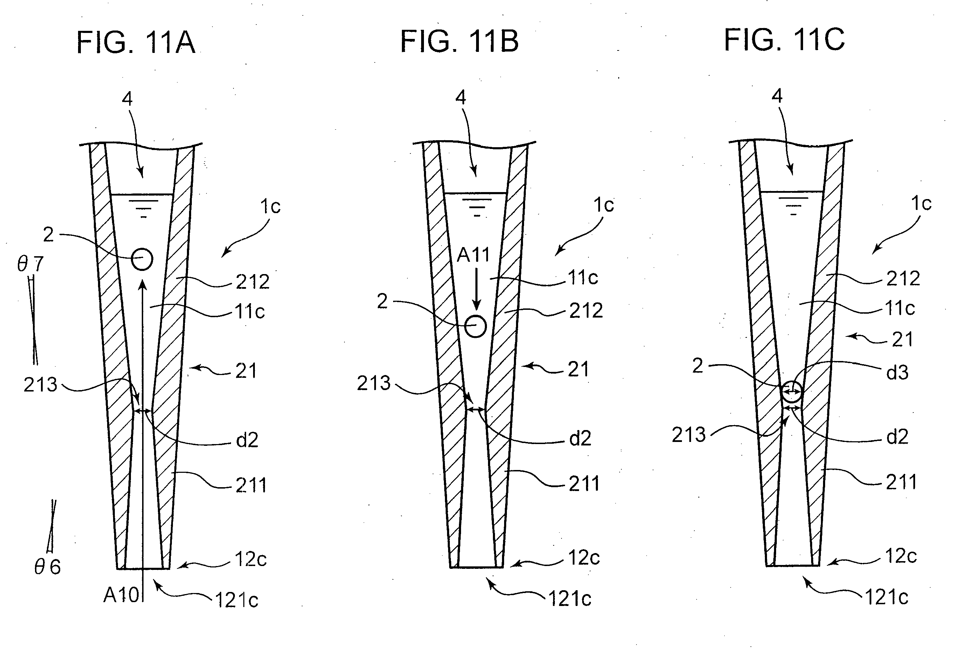

The ratio (diameter reduction ratio) at which the tapered portion 211 narrows the flow channel for the sucked cell aggregate 2 from upstream toward downstream in the suction direction may be fixed, or may be varied in such a manner that the diameter reduction ratio is gradually increased. Specifically, as will be described later referring to FIG. 11A, the tilt angle θ6 of the tapered portion 211 may be fixed or may be varied.

Preferably, the tilt angle θ6 may be not smaller than 2° but smaller than 90°. When the tilt angle θ6 is smaller than 2°, the distance from the suction port 121c to the through-hole 213 may be unduly increased. As a result, by the point of time when the suction operation is completed, the cell aggregate 2 may not be sucked downstream in the suction direction with respect to the through-hole 213, and the cell aggregate 2 may not be trapped. In view of the above, in the embodiment, the tilt angle θ6 is set to 10°.

The through-hole 213 is an opening formed in the other end of the tapered portion 211, and also serves as an opening formed in one end of the inverse tapered portion 212. The diameter d2 of the through-hole 213 is not specifically limited. It is possible to set the diameter d2 of the through-hole 213 to be slightly smaller than the diameter d3 of the cell aggregate 2 (see FIG. 11C to be described later). In the embodiment, the diameter d2 of the through-hole 213 is set to 270 µm, assuming that a cell aggregate 2 whose diameter d3 is 300 µm is sucked. The cell aggregate 2 that has reached the through-hole 213 may be slightly deformed by the suction force generated in the tubular passage 11 c during a suction operation, but passes through the through-hole 213 downstream in the suction direction.

The sectional shape of the through-hole 213 is not specifically limited. Examples of the sectional shape of the through-hole 213 may include various shapes such as a circular shape, an elliptical shape, and a polygonal shape. In the embodiment, the through-hole 213 has a circular shape.

The inverse tapered portion 212 is a member configured such that the through-hole 213 is an opening formed in one end of the inverse tapered portion 212, and a connection port 214 to be connected to the suction pipette 10 (see FIG. 1), which is an opening formed in the other end of the tubular passage 11c, is an opening formed in the other end of the inverse tapered portion 212 for expanding the flow channel of the tubular passage 11c downstream in the suction direction. The downstream end of the inverse tapered portion 212 in the suction direction is joined to the inner wall of the tubular passage 11c.

The ratio (diameter reduction ratio) at which the flow channel for the sucked cell aggregate 2 is expanded by the inverse tapered portion 212 from upstream toward downstream in the suction direction may be fixed, or may be varied in such a manner that the diameter reduction ratio is gradually increased. Specifically, as will be described later referring to FIG. 11A, the tilt angle θ7 of the inverse tapered portion 212 may be fixed or may be varied.

Preferably, the tilt angle θ7 may be not smaller than 2° but smaller than 90°. When the tilt angle θ7 is smaller than 2°, the distance from the through-hole 213 to the connection port 214 tends to be unduly increased. In view of the above, in the embodiment, the tilt angle θ7 is set to 10°. The tilt angle θ6 of the tapered portion 211 may be the same as the tilt angle θ7 of the inverse tapered portion 212, or may be different from the tilt angle θ7 of the inverse tapered portion 212.

The tapered portion 211 and the inverse tapered portion 212 may be integrally formed with the suction tip 1c, or may be joined to the inner wall of the suction tip 1c as individual members. In the embodiment, the tapered portion 211 and the inverse tapered portion 212 are integrally formed with the suction tip 1c. The method for integrally forming the tapered portion 211 and the inverse tapered portion 212 with the suction tip 1c is not specifically limited. For instance, it is possible to integrally form the tapered portion 211 and the inverse tapered portion 212 with the suction tip 1c by well-known molding methods such as injection molding.

FIGS. 11A to 11C are schematic diagrams illustrating a behavior of the cell aggregate 2 to be sucked by using the suction tip I c in the embodiment. As illustrated in FIG. 11A, when the cell aggregate 2 travels downstream in the suction direction through the through-hole 213, as illustrated in FIG. 11B, at a point of time when the suction operation is completed or the section force is weakened, the cell aggregate 2 sinks in the gravitational direction within the tubular passage 11c. During the sinking, the cell aggregate 2 travels upstream in the suction direction along an inverse tapered surface formed on the inner wall of the inverse tapered portion 212, and reaches the through-hole 213. The cell aggregate 2 that has reached the through-hole 213 simply falls in the gravitational direction, and is not deformed to such an extent that the cell aggregate 2 passes through the through-hole 213. Therefore, as illustrated in FIG. 11C, the cell aggregate 2 is trapped downstream of the through-hole 213 in the suction direction in a state that the cell aggregate 2 comes into contact with the inverse tapered surface of the inverse tapered portion 212. In FIG. 11A, the arrow A10 indicates a behavior of the cell aggregate 2 that is sucked through the suction port 121c and travels downstream of the through-hole 213 in the suction direction. In FIG. 11B, the arrow A11 indicates a behavior of the cell aggregate 2 falling upstream in the suction direction in the gravitational direction.

As described above, the suction tip 1c in the embodiment is configured such that the cell aggregate 2 is sucked along the tapered surface of the tapered portion 211, as necessary, and reaches the through-hole 213. The cell aggregate 2 passes through the through-hole 213 with slight deformation. When the cell aggregate 2 has passed through the through-hole 213, the cell aggregate 2 falls in the gravitational direction, and is trapped while coming into contact with the inverse tapered surface of the inverse tapered portion 212 at a position downstream of the through-hole 213 in the suction direction. Further, when the trapped cell aggregate 2 is observed by the object observation device 3 to be described later, the user is only required to observe a position downstream of the through-hole 213 in the suction direction. This eliminates the need of searching the cell aggregate 2, and allows for the user to easily check the shape of the trapped cell aggregate 2.

### <Object Observation Device>

### (Fifth Embodiment)

In the following, the object observation device 3 in the fifth embodiment of the invention is described in detail referring to the drawings. FIG. 12 is a schematic diagram illustrating a configuration of the object observation device 3 in the embodiment.

The object observation device 3 in the embodiment includes the Petri dish 31 (vessel including the inner bottom portion 311, and configured to store the liquid 4 containing the cell aggregate 2 (object) to be sucked); a stage 32 for placing the Petri dish 31 thereon; the suction tip 1 as a jig for sucking the cell aggregate 2; the suction pipette 10 (suction means) connected to the suction tip 1, and configured to generate a suction force for sucking the object; the imaging device 33 (observing means provided with an optical lens system) for capturing an image of the cell aggregate 2 to be trapped in the horizontal portion 14 (trap portion) within the depth of field of the optical lens system; and a moving device 34 (driving means) for moving the suction pipette 10.

As described in detail in the first embodiment, the suction tip 1 is internally provided with the tubular passage 11 serving as a suction path for sucking the cell aggregate 2. The suction tip 1 is provided with the distal end portion 12 oriented in a substantially vertical direction when in use and including the suction port 121 for sucking the cell aggregate 2, the suction port 121 being an opening formed in one end of the tubular passage 11; the converting portion 13 continuously formed with the distal end portion 12, and configured to convert the extending direction of the tubular passage 11 in the distal end portion 12 in a horizontal direction; the horizontal portion 14 (trap portion) formed downstream of the converting portion 13 in the suction direction, and configured to trap the cell aggregate 2; the re-converting portion 15 formed downstream of the horizontal portion 14 in the suction direction, and configured to re-convert the extending direction of the tubular passage 11 in a substantially vertical direction; and the main body portion 16 formed downstream of the re-converting portion 15 in the suction direction and including the connection port 161 to be connected to the suction pipette 10 (the suction means), the connection port 161 being an opening formed in the other end of the tubular passage 11.

The Petri dish 31 and the cell aggregate 2 (object) have the same configurations as the configurations described in detail in the first embodiment, and therefore, repeated description thereof is omitted.

The stage 32 is a horizontally flat plate-like member provided with a rectangular holder (not illustrated) for holding the Petri dish 31 thereon. On the stage 32, there is provided a position adjustment mechanism (not illustrated) for moving the Petri dish 31 manually or automatically in three directions i.e. front and right directions, left and right directions, and up and down directions. When it is possible to capture an image of the cell aggregate 2 held in the Petri dish 31 within the depth of field of an objective lens 331 by moving the objective lens 331 provided in the imaging device 33 to be described later up and down, a mechanism in the position adjustment mechanism for moving the Petri dish 31 up and down is not an essential element. As far as the position adjustment mechanism has a mechanism for moving the Petri dish 31 in left and right directions, the above configuration is implementable. By the position adjustment mechanism, the position of the Petri dish 31 placed on the stage 32 is adjusted so that the position of the held cell aggregate 2 to be sucked lies within the observable range of the imaging device 33 to be described later. As described above, observing the cell aggregate 2 held in the Petri dish 31 from below the Petri dish 31 using the imaging device 33 makes it possible to observe the position and the shape of the cell aggregate 2 held in the liquid 4 stored in the Petri dish 31, and makes it easy to move the suction port 121 of the suction tip 1 to a position where it is easy to suck the cell aggregate 2 by operating the moving device 34 to be described later.

The suction pipette 10 (suction means) is a tubular member capable of generating a suction force. The suction pipette 10 is allowed to suck the cell aggregate 2 from the suction port 121, which is an opening formed in one end of the tubular member 11 of the suction tip 1, by generating a suction force in the tubular passage 101 (see FIG. 1) of the suction pipette 10 in a state that the suction pipette 10 is connected to the suction tip 1. The suction pipette 10 is used by being connected to the moving device 34 to be described later. Driving of the suction pipette 10 is controlled by the moving device 34, and the suction pipette 10 is moved up and down.

The imaging device 33 (observing means provided with an optical lens system) is a device which is disposed below the Petri dish 31 for placing the stage 32 thereon, and is configured to observe the cell aggregate 2 held in the Petri dish 31 and the cell aggregate 2 to be trapped in the horizontal portion 14 of the suction tip 1 from below. The imaging device 33 is provided with the objective lens 331, an unillustrated diaphragm of the objective lens 331, a field stop of an eyepiece lens, an eyepiece lens, a CCD (Charge Coupled Device) image sensor as an imaging element, an image processing unit, and a display device 332. An optical lens system such as the objective lens 331 included in the imaging device 33 is disposed at such a position that not only an image of the cell aggregate 2 to be trapped in the horizontal portion 14 but also an image of the cell aggregate 2 held in the Petri dish 31 are captured within the depth of field. In view of the above, with use of the optical lens system as described above, the user is allowed to sequentially check the position and the shape of the cell aggregate 2 in the Petri dish 31 before a suction operation, and to check the position and the shape of the cell aggregate 2 trapped in the horizontal portion 14 after the suction operation. This is advantageous in enhancing work efficiency. The CCD image sensor converts a light image formed on a light receiving surface of the sensor into an electrical image data signal. The image processing unit performs image processing such as gamma correction or shading correction to image data, as necessary. The display device 332 displays image data after the image processing. Thus, the user is allowed to observe an image displayed on the display device 332.

The position of the imaging device 33 is not specifically limited. The imaging device 33 may be disposed above the Petri dish 31, in place of being disposed below the Petri dish 31. In the embodiment, the imaging device 33 is disposed below the Petri dish 31, taking into consideration the layout of the devices including the object observation device 3.

The moving device 34 (driving means) is a device for holding the suction pipette 10 and for moving the held suction pipette 10 up and down. The moving device 34 is provided with a main body portion 341 to be connected to the suction pipette 10, and a guide portion 342 along which the main body portion 341 runs. The main body portion 341 is provided with a motor (not illustrated) for moving the suction pipette 10 up and down by moving the main body portion 341 in up and down directions within a substantially rectangular parallelepiped housing, a controller (not illustrated) for controlling the motor, and a syringe pump (not illustrated) for generating a suction force. A connection port (not illustrated), as a suction port, to be connected to the suction pipette 10 is formed in the outer portion of the housing of the main body portion 341 for generating a suction force by the syringe pump. A linear gear (rack gear) is formed on the guide portion 342. A circular gear (pinion gear) is formed on the main body portion 341. Driving the motor to be controlled by the controller allows for the main body portion 341 to run along the guide portion 342. The motor is capable of calibrating the object observation device 3 by moving the main body portion 341 in front and rear directions and in left and right directions, in addition to up and down directions so that an image of the suction port 121 of the suction tip 1 can be captured within the depth of field of the objective lens 331 of the imaging device 33. Calibration is performed, as necessary, at the time of replacing the suction tip 1 or at the time of starting up the device.

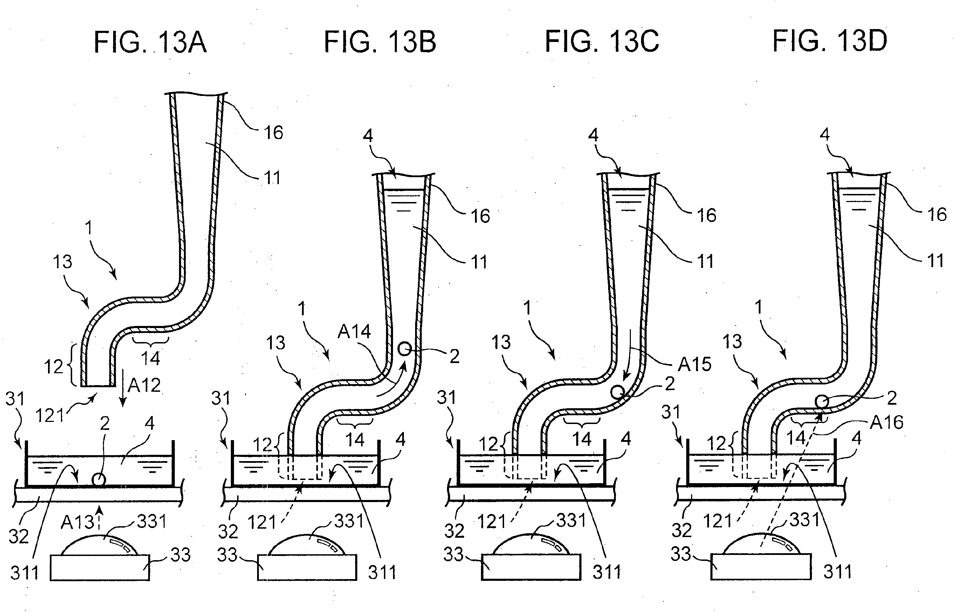

FIGS. 13A to 13D are schematic diagrams illustrating an operation of sucking the cell aggregate 2 and observing the sucked cell aggregate 2 by using the object observation device 3 in the embodiment of the invention.

As illustrated in FIG. 13A, when the main body portion 341 is moved down, the distal end portion 12 of the suction tip 1 is inserted in the Petri dish 31 in which the cell aggregate 2 is held, whereby the suction port 121 comes close to the cell aggregate 2. The images of the position of the cell aggregate 2 and of the position of the suction port 121 are displayed on the display device 332 of the imaging device 33. Therefore, the user is allowed to accurately move the suction port 121 close to the cell aggregate 2, while viewing the positions.

As illustrated in FIG. 13B, when a suction force is generated in the tubular passage 11 of the suction tip 1 by the suction pipette 10, the cell aggregate 2 is sucked into the tubular passage 11. The sucked cell aggregate 2 travels downstream in the suction direction within the tubular passage 11, and reaches the main body portion 16 via the converting portion 13, the horizontal portion 14 (trap portion), and the re-converting portion 15 described in detail in the first embodiment.

As illustrated in FIG. 13C, when the suction operation is completed, the cell aggregate 2 starts to sink in the gravitational direction, and travels upstream in the suction direction. Then, as illustrated in FIG. 13D, the cell aggregate 2 is trapped in the horizontal portion 14 in a stationary state. The cell aggregate 2 trapped in the horizontal portion 14 is observed by the imaging device 33. In order to securely capture an image of the cell aggregate 2 trapped in the horizontal portion 14 within the depth of field of the objective lens 331, the imaging device 33 itself may be moved in front and rear directions and in left and right directions so as to move the objective lens 331 at a position below the horizontal portion 14; or the objective lens 331 may be moved up and directions for focusing. In FIG. 13A, the arrow A12 indicates a moving direction of the distal end portion 12, and the arrow A13 indicates an observing direction of the imaging device 33 for observing the cell aggregate 2 held in the Petri dish 31. In FIG. 13B, the arrow A 14 indicates a behavior of the cell aggregate 2 that is sucked through the suction port 121 and travels to the main body portion 16. In FIG. 13C, the arrow A15 indicates a behavior of the cell aggregate 2 falling upstream in the suction direction in the gravitational direction. In FIG. 13D, the arrow A16 indicates an observing direction of the imaging device 33 for observing the cell aggregate 2 trapped in the horizontal portion 14.

When a suction tip (see FIGS. 22A to 22C) provided with a linear tubular passage extending from a suction port is used as in a conventional art, it is necessary to take out the suction port from the liquid so as not to discharge the cell aggregate through the suction port. Further, after the suction port is taken out from the liquid, the cell aggregate continues to fall in the gravitational direction toward the suction port through the linear tubular passage. As a result, it is difficult to accurately observe the moving cell aggregate while focusing on the moving cell aggregate. In view of the above, in the conventional art, after the suction port is taken out from the liquid, it is necessary to wait for a while until the cell aggregate finishes sinking and is kept in a stationary state near the suction port, and then to observe the vicinity of the suction port; or it is necessary to eject the sucked cell aggregate onto a separately prepared collection plate, and to observe the cell aggregate in the collection plate. Further, in the case where the vicinity of the suction port is observed after the suction port is taken out from the liquid, the distance between the objective lens and the suction port increases. This may make it difficult to capture an image of the cell aggregate within the depth of field, even if the objective lens is moved to the maximum limit in up and down directions. Contrary to the above, the object observation device 3 in the embodiment is capable of performing the operations of sucking, trapping, and observing the cell aggregate 2 in a state that the suction port 121 is immersed in the liquid 4. This is advantageous in shortening the time required for taking out the suction port 121 from the liquid 4. Further, the horizontal portion 14 is formed downstream of the suction portion 121 in the suction direction. Therefore, the time required for the sucked cell aggregate 2 to be trapped in the horizontal portion 14 is shorter than the time required for the cell aggregate to sink in the suction port and be trapped, as in the conventional art. This makes it possible to shorten the time required from finishing a suction operation to starting an observation operation. Further, it is not necessary to observe the cell aggregate 2 after the cell aggregate 2 is ejected onto the collection plate. Thus, the object observation device 3 is advantageous in remarkably shortening the time required from a suction operation to an observation operation.

As described above, the object observation device 3 in the embodiment is configured such that the suction tip 1 is connected to the distal end of the suction pipette 10. The movement of the suction pipette 10 is controlled by the moving device 34. The suction port 121 of the suction tip 1 is formed in one end of the distal end portion 12 disposed in a substantially vertical direction when in use. According to this configuration, when the suction pipette 10 is moved down by the moving device 34, the suction port 121 comes sufficiently close to the cell aggregate 2 held in the liquid 4 stored in the Petri dish 31 in a substantially vertical direction. Further, when a suction force is generated within the tubular passage 11 by the suction pipette 10 in the above state, it is possible to suck the cell aggregate 2 through the suction port 121. The sucked cell aggregate 2 is trapped in the horizontal portion 14 of the suction tip 1 in a stationary state. Therefore, the cell aggregate 2 is not discharged through the suction port 121 even when the suction port 121 is immersed in the liquid 4. Further, an image of the cell aggregate 2 trapped in the horizontal portion 14 is captured within the depth of field of the optical lens system provided in the imaging device 33, without moving the imaging device 33, or by slightly moving the objective lens up and down. This makes it easy for the user to check whether the cell aggregate 2 is trapped in the horizontal portion 14. Further, in the object observation device 3 in the embodiment, it is possible to observe the horizontal portion 14 by the imaging device 33 in a state that the suction port 121 is immersed in the liquid 4. This is advantageous in remarkably shortening the time required from a suction operation to an observation operation.

In particular, the optical lens system provided in the imaging device 33 is configured to capture not only an image of the cell aggregate 2 trapped in the horizontal portion 14 but also an image of the cell aggregate 2 held in the Petri dish 31 within the depth of field, without moving the imaging device 33, or by slightly moving the objective lens up and down. This allows for the user to check whether the cell aggregate 2 is accurately sucked from the Petri dish 31 and then to check the horizontal portion 14 for checking whether a collection operation is successful; and to promptly check whether the cell aggregate 2 is deformed or not during a suction operation by performing the suction operation while observing the cell aggregate 2 held in the Petri dish 31. This is advantageous in enhancing work efficiency.

### <Object Observing Method>

### (Sixth Embodiment)

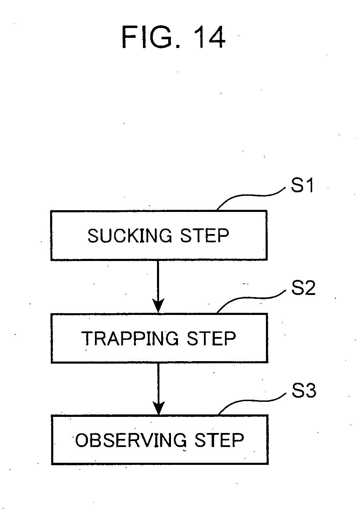

In the following, an object observing method in the sixth embodiment of the invention is described in detail referring to the drawings. FIG. 14 is a flowchart describing the steps of the object observing method in the embodiment. The object observing method in the embodiment includes a sucking step (S1), a trapping step (S2), and an observing step (S3). The object observing method is performed using the object observation device 3 (see FIG. 12) described in detail in the fifth embodiment, for instance. In view of the above, in the following description, a case in which the object observing method in the embodiment is performed using the aforementioned object observation device 3, is exemplified.

The user determines a cell aggregate 2 to be sucked before performing a suction operation. Specifically, out of the cell aggregates 2 within the Petri dish 31, a cell aggregate 2 whose shape or the like is appropriate for the purpose of use is determined by visual observation or by judging an image processed by an image processing program. An observation operation is performed by adjusting the optical lens system of the imaging device 33 to such a position that an image of the cell aggregate 2 in the Petri dish 31 is captured within the depth of field of the optical lens system, and by judging an image (including a moving image) displayed on the display device 332 provided in the imaging device 33 by visual observation or by judgment with use of an image processing program. After the cell aggregate 2 to be sucked is determined, the sucking step (S1) is carried out.

The sucking step (S1) is a step of sucking the cell aggregate (object) by the suction pipette 10 (suction means) connected to the suction tip 1. The sucking step is described referring to FIG. 13A.

As described in detail in the first embodiment, the suction tip 1 is internally provided with the tubular passage 11 serving as a suction path for sucking the cell aggregate 2. The suction tip 1 is provided with the distal end portion 12 disposed in a substantially vertical direction when in use and including the suction port 121 for sucking the cell aggregate 2, the suction port 121 being an opening formed in one end of the tubular passage 11; the converting portion 13 continuously formed with the distal end portion 12, and configured to convert the extending direction of the tubular passage 11 in the distal end portion 12 in a horizontal direction; the horizontal portion 14 (trap portion) formed downstream of the converting portion 13 in the suction direction, and configured to trap the cell aggregate 2; the re-converting portion 15 formed downstream of the horizontal portion 14 in the suction direction, and configured to re-convert the extending direction of the tubular passage 11 in a substantially vertical direction; and the main body portion 16 formed downstream of the re-converting portion 15 in the suction direction and including the connection port 161 to be connected to the suction pipette 10 (suction means), the connection port 161 being an opening formed in the other end of the tubular passage 11.

The cell aggregate 2 is held in the Petri dish 31 (vessel) storing the liquid 4. The distal end portion 12 including the suction port 121 of the suction tip 1 is disposed in a substantially vertical direction at a position above the Petri dish 31. The suction tip 1 is connected to the suction pipette 10. The suction pipette 10 is connected to the moving device 34 (see FIG. 12).

When the main body portion 341 of the moving device 34 is moved down, the distal end portion 12 of the suction tip 1 is inserted in the Petri dish 31 holding the cell aggregate 2 therein, whereby the suction port 121 comes close to the cell aggregate 2. When a suction force is generated within the tubular passage 11 of the suction tip 1 by the suction pipette 10, the cell aggregate 2 is sucked into the tubular passage 11. Judgment as to whether the suction operation is successful is made, referring to an image displayed on the display device 332 (see FIG. 12).

The trapping step (S2) is a step of trapping the sucked cell aggregate 2 in the horizontal portion 14. The trapping step is described referring to FIG. 13B and FIG. 13C. The cell aggregate 2 sucked in the sucking step travels downstream in the suction direction along with the sucked liquid 4 by a suction force generated in the tubular passage 11 via the converting portion 13, the horizontal portion 14 (trap portion), and the re-converting portion 15; and reaches the main body 16.

When the suction operation is completed, the cell aggregate 2 starts to sink in the gravitational direction. The cell aggregate 2 travels upstream in the suction direction from the main body portion 16 to the horizontal portion 14 via the re-converting portion 15, and is trapped in the horizontal portion 14 in a stationary state.

The observing step (S3) is a step of observing the trapped cell aggregate 2. The observing step is described referring to FIG. 13D. An observation operation is performed by observing the horizontal portion 14 by the imaging device 33. By observing the horizontal portion 14, it is possible to check whether the cell aggregate 2 is deformed or not during a suction operation, in addition to judgment as to whether the collection operation is successful.

After checking that the cell aggregate 2 is appropriately sucked, moving the main body portion 341 (see FIG. 12) up makes it possible to move the suction pipette 10 up, whereby the distal end portion 12 including the suction port 121 is taken out from the liquid 14 stored in the Petri dish 31. The cell aggregate 2 trapped in the horizontal portion 14 is ejected onto the collection plate (not illustrated) disposed adjacent to the Petri dish 31 on the stage 32 for preservation or for various tests.

As described above, according to the object observing method in the embodiment, the sucking step of sucking the cell aggregate 2 is followed by the trapping step of trapping the sucked cell aggregate 2 in the horizontal portion 14 in a stationary state. Therefore, it is possible to prevent the sucked cell aggregate 2 sank in the gravitational direction discharging of the sucked cell aggregate 2 through the suction port 121, even when the suction port 121 is immersed in the liquid 4. Further, the object observing method includes the observing step of observing the cell aggregate 2 trapped in the horizontal portion 14. This makes it possible to check the shape of the cell aggregate 2, in addition to judgment as to whether the suction operation of the cell aggregate 2 is successful.

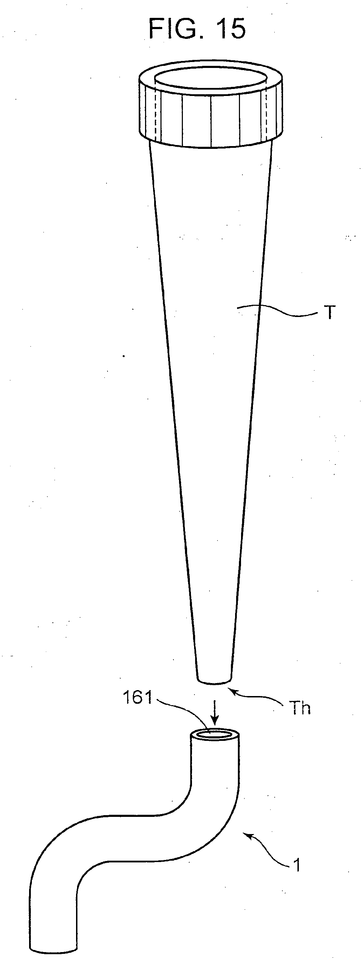

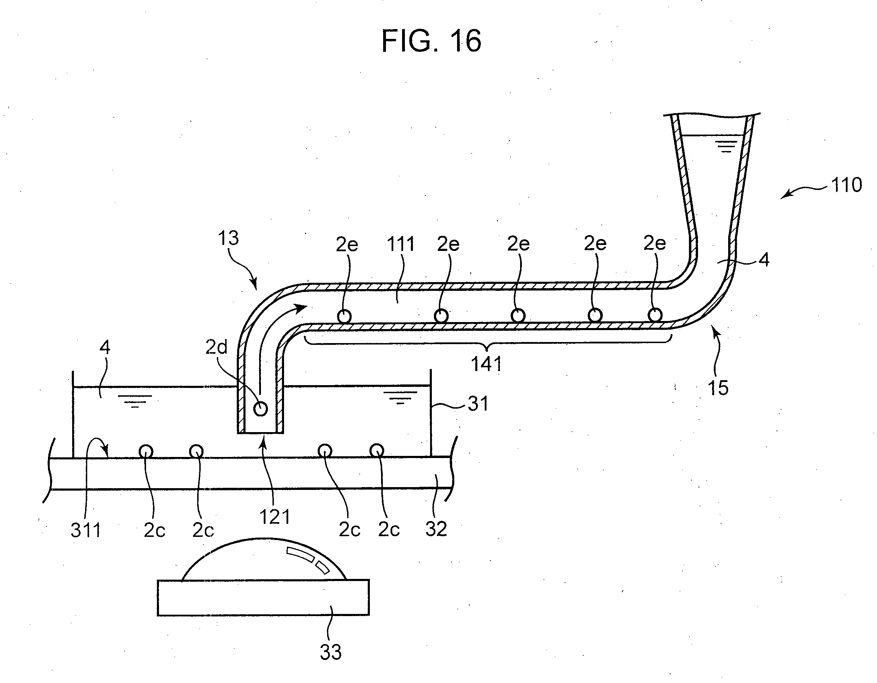

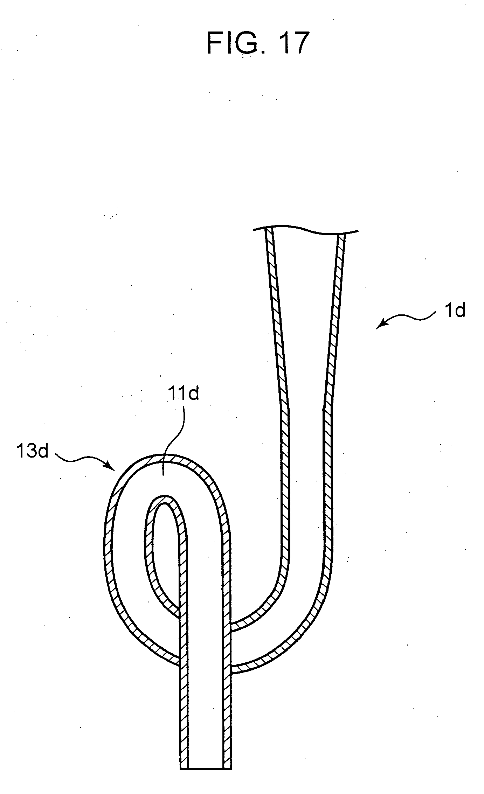

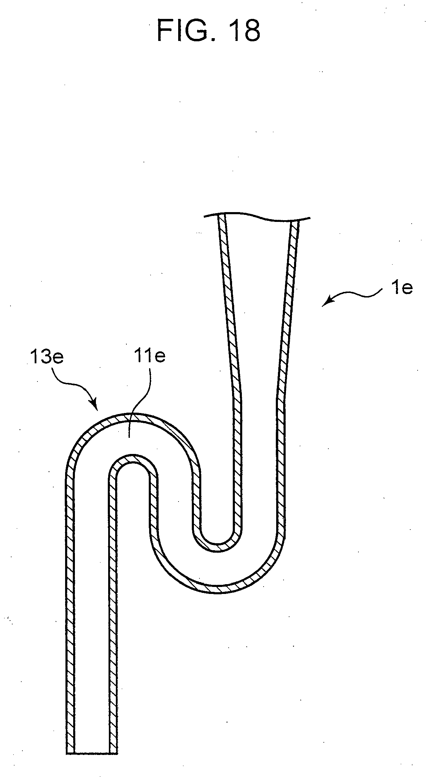

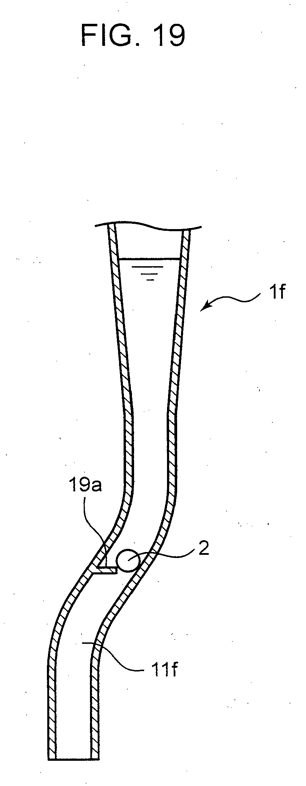

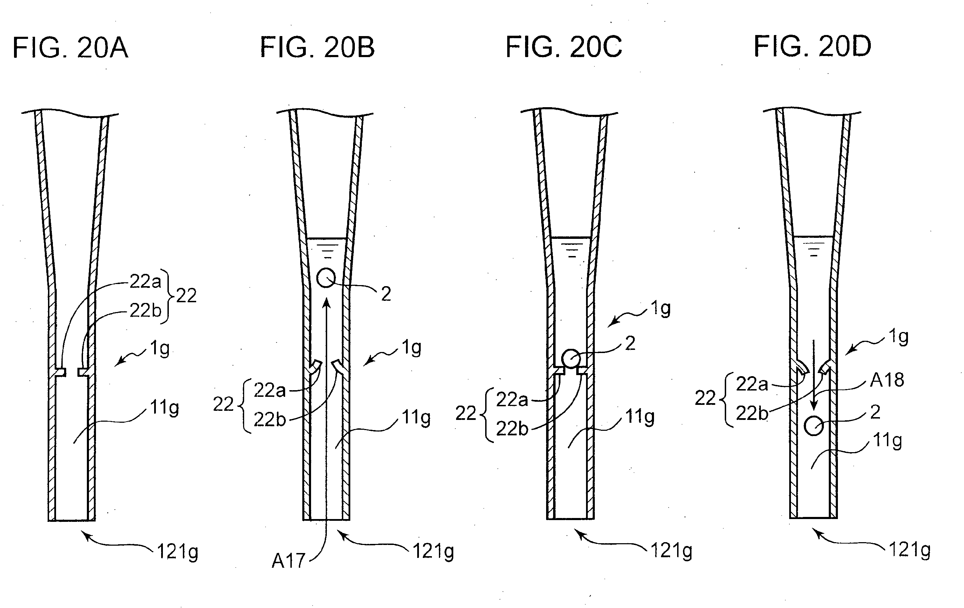

The embodiments of the invention have been described as above. The invention, however, is not limited to the above, and the following modified embodiments may be applied.
(1) In the embodiment, a suction tip has a connection port formed in a main body portion or in a trap portion to be connected to a suction pipette (suction means). Alternatively, in the invention, as illustrated in FIG. 15, a connection port 161 of a suction tip 1 may be connected to a well-known suction tip T for use. FIG. 15 is a schematic diagram illustrating a case, in which the inventive suction tip is used as a jig to be connected to a well-known suction tip. In this case, the inventive suction tip 1 is used as a jig to be connected to a suction port Th of the well-known suction tip T.
(2) In the embodiment, an object to be trapped in a trap portion is one object. Alternatively, in the invention, two or more objects may be simultaneously or sequentially sucked through a suction port, and trapped in a trap portion. FIG. 16 is a schematic diagram illustrating a behavior of two or more cell aggregates (objects) to be sucked using another example of the suction tip 1 in the first embodiment (suction tip 110). The suction tip 110 has substantially the same configuration as the suction tip 1 described in detail in the first embodiment except that the horizontal length of a horizontal portion 141 is longer than the horizontal length of the horizontal portion 14 of the suction tip 1 in the first embodiment, and therefore, repeated description thereof is omitted.
   A liquid 4 is stored in a Petri dish 31 (vessel). Two or more cell aggregates are held on an inner bottom portion 311 of the Petri dish 31. The reference sign 2c denotes cell aggregates held on the inner bottom portion 311. A suction port 121 of the suction tip is immersed in the liquid 4. When a suction force is generated in a tubular passage 111 by a suction pipette (not illustrated) connected to the suction tip 110 in this state, the cell aggregates 2c held on the inner bottom portion 311 are successively sucked into the tubular passage from the cell aggregate 2c closest to the suction port 121. The reference sign 2d denotes cell aggregates that are sucked through the suction port 121 and travel downstream in the suction direction.
   Alternatively, a stage 32 carrying the Petri dish 31 or a suction pipette connected to the suction tip 110 may be moved so that the suction port 121 comes close to a position above the cell aggregates 2c held on the inner bottom portion 311 in order to easily suck the cell aggregates 2c held on the inner bottom portion 311. Further, in place of sucking two or more cell aggregates by a one-time suction operation, it is possible to suck one or more cell aggregates, followed by temporarily stopping the suction operation, and to resume the suction operation after the suction port 121 is moved to a position above the cell aggregates 2c held on the inner bottom portion 311. The number of cell aggregates to be sucked and trapped in the horizontal portion 141 is not specifically limited. The number may be determined, as necessary, taking into consideration the length of the horizontal portion 141, the size of the cell aggregates, or the capacity of a collection plate (not illustrated).
   When the suction operation is completed or the suction force is weakened, the sucked cell aggregates sink in the horizontal portion 141 in the gravitational direction, and are trapped in the tubular passage of the horizontal portion 141. In this state, the previously sucked cell aggregates travel downstream in the suction direction with respect to the cell aggregates that are sucked lately. Therefore, the previously sucked cell aggregates are trapped at a position downstream of the horizontal portion 141 in the suction direction. As a result, the sucked two or more cell aggregates are trapped in the horizontal portion to be away from each other. The reference sign 2e denotes cell aggregates trapped in the horizontal portion 141 to be away from each other. The held cell aggregates 2e are individually observed by an imaging device 33 disposed below the stage 32. Thereafter, when a flow of liquid for ejecting the objects upstream in the suction direction is generated by the suction pipette, the cell aggregates 2e are successively ejected onto a collection plate (not illustrated) in the order from the cell aggregate 2e trapped at a most upstream position in the suction direction. When the trapped cell aggregates include a cell aggregate that is not sucked (e.g. a cell aggregate 2a having a distorted shape as illustrated in FIG. 23), the distorted cell aggregate is ejected onto a plate other than the collection plate for discrimination.
   In FIG. 16, the cell aggregates 2e are trapped in a horizontal direction to be away from each other. In another example, the interval between the held cell aggregates 2e is not specifically limited. Two or more cell aggregates 2e may be trapped without an interval. A train of trapped cell aggregates 2e may be simultaneously ejected, or may be grouped by generating a flow of liquid within the tubular passage, as necessary, so that the cell aggregates 2e are ejected group by group.
   According to this example, it is possible to eject two or more cell aggregates after sucking and observing the cell aggregates individually. This is advantageous in enhancing work efficiency, as compared with a case, in which cell aggregates are sucked, observed, and ejected one by one.
(3) In the embodiment (second embodiment), a suction tip includes a converting portion for converting the extending direction of a tubular passage by the angle θ1. Alternatively, in the invention, as illustrated in FIG. 17, a suction tip 1d may include a converting portion 13d obtained by converting the extending direction of a tubular passage 11d by one turn, or as illustrated in FIG. 18, a suction tip 1e may include a converting portion 13e obtained by converting the extending direction of a tubular passage 11e into a U-shape. FIG. 17 and FIG. 18 are schematic diagrams illustrating other examples of the converting portion 13a of the suction tip 1a in the second embodiment. Applying the converting portion 13d or the converting portion 13e to the inventive suction tip makes it possible to securely prevent a cell aggregate from traveling upstream in the suction direction and from sinking beyond the suction port after reaching the distal end portion.
(4) In the embodiment (third embodiment), a suction tip is provided with a projection piece axially projecting from the inner wall of a tubular passage. One end of the projection piece is joined to the lower portion of the inner wall of the tubular passage in the trap portion, and the other end thereof is a free end. Alternatively, in the invention, as illustrated in FIG. 19, a suction tip If may be provided with a projection piece 19a axially projecting from the inner wall of a tubular passage, wherein one end of the projection piece is joined to the upper portion of the inner wall of the tubular passage in the trap portion, and the other end thereof is a free end. FIG. 19 is a schematic diagram illustrating another example of the projection piece 19 provided in the suction tip 1b in the third embodiment. In this case, the free end of the projection piece 19a and the lower portion of the inner wall of a tubular passage 11f can restrain the cell aggregate 2 from traveling upstream in the suction direction for trapping. Further, a projection piece may be provided on the horizontal portion 14 (see FIG. 1 to FIG. 3) exemplified in the first embodiment. Further, the number of projection pieces is not limited to one, but may be two or more.
(5) In the embodiment (fourth embodiment), a suction tip is constituted of a distal end portion and a reduced diameter portion (trap portion), and is not provided with a main body portion. Alternatively, in the invention, a suction tip may be further provided with a cylindrical main body portion formed downstream of the diameter reduced portion in the suction direction, and an opening of the main body portion formed downstream in the suction direction may serve as a connection port to be connected to a suction pipette (suction means). Further, in the embodiment (fourth embodiment), a distal end portion of a suction tip is not provided with a tubular passage of a predetermined length, and an opening formed in one end of a tapered portion functions as a suction port for sucking a cell aggregate. Alternatively, in the invention, a suction tip may be provided with a cylindrical distal end portion continuing to the opening formed in one end of the tapered portion, and an opening formed upstream of the distal end portion in the suction direction may serve as a suction port.
(6) In the embodiment (fourth embodiment), a suction tip has a through-hole in one end of a tapered portion for passing a cell aggregate therethrough. Alternatively, in the invention, as illustrated in FIGS. 20A to FIG. 20D, a suction tip 1g may be configured such that a valve member 22 constituted of a pair of axially projecting valves (a valve 22a and a valve 22b), each of which has one end joined to the inner wall of a tubular passage 11g, is provided for trapping a cell aggregate 2 in a stationary state by the valve member 22. FIGS. 20A to 20D are schematic diagrams illustrating a behavior of a cell aggregate 2 to be sucked using another example of the suction tip in the fourth embodiment (suction tip 1g).
   As illustrated in FIG. 20A, free ends of the valve 22a and the valve 22b constituting the valve member 22 are away from each other by a distance smaller than the diameter of the cell aggregate 2 in an unused state (a state that a suction force is not generated in the tubular passage 11g). On the other hand, as illustrated in FIG. 20B, during a suction operation, the valve 22a and the valve 22b are flexed downstream in the suction direction due to a flow of liquid to be sucked together with the cell aggregate 2, and a gap of a size capable of passing the cell aggregate 2 is formed. As illustrated in FIG. 20C, at a point of time when the suction operation is completed or the suction force is weakened, the valve 22a and the valve 22b return to the unused state. Therefore, the cell aggregate 2 falling in the gravitational direction is trapped in a stationary state while coming into contact with the valve member 22 at a position downstream (trap portion) of the valve member 22 in the suction direction. On the other hand, during an ejection operation, as illustrated in FIG. 20D, the valve 22a and the valve 22b are flexed upstream in the suction direction due to a flow of liquid to be ejected, and a gap of a size capable of passing the cell aggregate 2 is formed. In FIG. 20B, the arrow A17 indicates a behavior of the cell aggregate 2 that is sucked through the suction port 121g during a suction operation, and travels downstream in the suction direction through the gap formed by flexing of the valve 22a and the valve 22b downstream in the suction direction. In FIG. 20D, the arrow A18 indicates a behavior of the cell aggregate 2 traveling upstream in the suction direction through the gap formed by flexing of the valve 22a and the valve 22b upstream in the suction direction during an ejection operation.
   According to the suction tip 1g having the above configuration, the cell aggregate 2 passes through the gap formed by flexing of the valve 22a and the valve 22b during a suction operation, and travels downstream of the valve member 22 in the suction direction. The valve member 22 is configured to form a gap which disables to pass the cell aggregate 2 in an unused state. Therefore, the cell aggregate 2 that has passed the valve member 22 falls in the gravitational direction, and is trapped in a stationary state while coming into contact with the valve member 22 at a position downstream of the valve member 22 in the suction direction. During an ejection operation, the cell aggregate 2 passes the gap formed by flexing of the valve 22a and the valve 22b, and travels upstream in the suction direction. Further, when the trapped cell aggregate 2 is observed by the aforementioned object observation device, the user is only required to observe a position downstream of the valve member 22 in the suction direction. This eliminates the need of searching the cell aggregate, and makes it easy for the user to check the shape of the trapped cell aggregate.
(7) In the embodiment (fourth embodiment), a suction tip includes a tapered portion and an inverse tapered portion on the inner wall of a trap portion, and a cell aggregate passes through a through-hole formed in a portion where the tapered portion and the inverse tapered portion are communicated, while deforming. Alternatively, in the invention, a tapered portion and an inverse tapered portion may be made of a flexible resin (flexible material) which is deformable by a stress to be exerted from the cell aggregate for expanding the diameter of the through-hole, when the cell aggregate passes through the through-hole. The flexible resin is not specifically limited. For instance, it is possible to use soft silicone, elastomer, PVDF (polyvinylidene fluoride), PVC (polyvinyl chloride), TPO (olefin-based elastomer), SBC (styrene-based elastomer), EVA (vinyl acetate), or soft rubber. This is advantageous in reducing a stress applied to the cell aggregate when the cell aggregate passes through the through-hole, whereby the object is less likely to be damaged by deformation or the like.
(8) In the embodiment (particularly, first to third embodiments), a suction tip includes a linear tubular passage of a predetermined length (e.g. a tubular passage included in the horizontal portion 14 of the suction tip 1 described in detail in the first embodiment) between a converting portion and a re-converting portion. Alternatively, in the invention, a tubular passage disposed between a converting portion and a re-converting portion may have a curved shape.

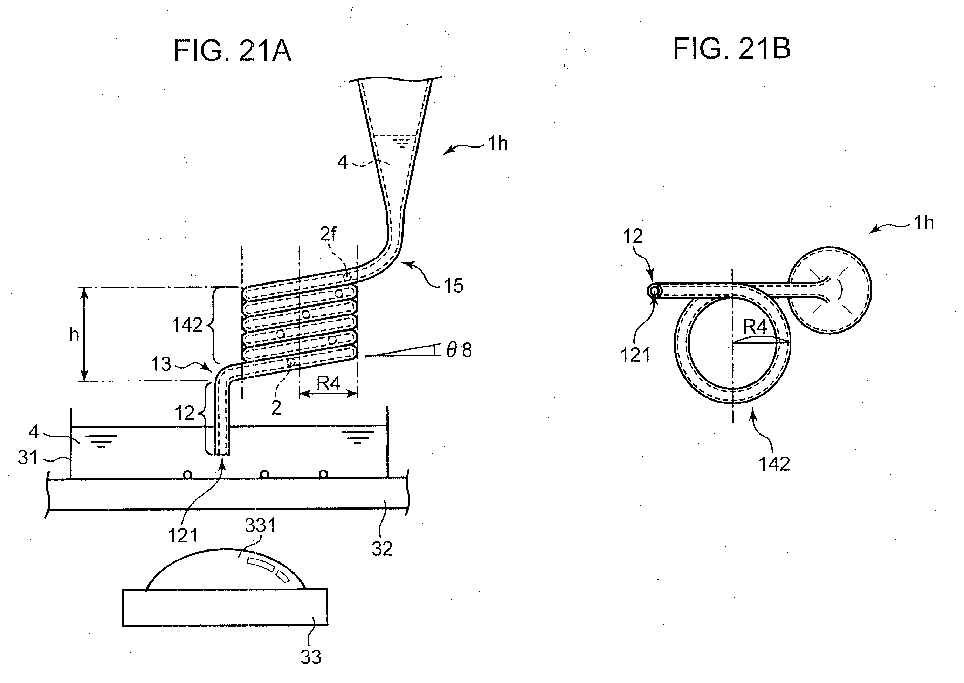

   FIGS. 21A and 21B are schematic diagrams of a suction tip 1h, as another example of the suction tip 1 in the first embodiment. The suction tip 1h is provided with a spiral portion 142 (trap portion, horizontal portion) including a spirally curved tubular passage. FIG. 21A is a side view of the suction tip 1h, and FIG. 21B is a bottom view of the suction tip 1h. As illustrated in FIG. 21A, the spiral portion 142 has one end connected to a converting portion 13, and the other end connected to a re-converting portion 15. The spiral portion 142 is disposed in such a manner that the axial direction of the spiral portion 142 is aligned with a vertical direction. The tubular passage of the spiral portion 142 is formed into a spiral shape with a moderate inclination (tilt angle θ8). According to this configuration, when a suction operation is completed and a flow of liquid is not generated in the tubular passage, a cell aggregate 2 sunk in the spiral portion 142 is trapped without traveling upstream in the suction direction through the spiral portion 142 by the weight thereof. The magnitude of the tilt angle θ8 is not specifically limited. As far as a cell aggregate does not travel by the weight thereof and is held stationary, the magnitude of the tilt angle θ8 may be in the range of from -3 to 15°, for instance. In the embodiment, the tilt angle θ8 is set to 3°. When the tilt angle θ8 is larger than 15°, the cell aggregate 2 may not be trapped in the spiral portion 142, may keep on sinking by the weight thereof, and may be ejected through a suction port 121.
   The number of windings of the tubular passage of the spiral portion 142 is not specifically limited. The number of windings may be determined, as necessary, taking into consideration the quantity of a liquid 4 to be sucked, or the number of cell aggregates 2 to be held. In this example, the spiral portion 142 has the number of windings of 6. The size (radius R4) of the spiral portion 142 is not specifically limited, and may be in the range of from about 0.5 to 20 mm. Further, the radius R4 may be fixed, or may be varied, as necessary. In the embodiment, the spiral portion 142 has the radius R4 of 2 mm (fixed). The spiral shape of the spiral portion 142 is not specifically limited. The spiral shape may be a circular shape or an elliptical shape. In this example, the spiral portion 142 has a circularly spiral shape.
   The height h of the spiral portion 142 is not specifically limited. The height h may be set to any value, as far as it is possible to capture an image of the cell aggregate 2 trapped in the spiral portion 142 within the depth of field of the objective lens 331 of the imaging device 33 disposed below the stage 32. Specifically, in FIG. 21A, the height h may be such that it is possible to capture an image of the cell aggregate (indicated by the reference sign 2f) held near the connecting portion between the spiral portion 142 and the re-converting portion 15 within the depth of field of the objective lens 331.
   The material of the spiral portion 142 is not specifically limited. The spiral portion 142 may be made of the same material as the material of another portion (e.g. the distal end portion 12) of the suction tip 1h, or may be connected to another portion (e.g. the distal end portion 12) using a material having a high degree of transparency (e.g. a material for optical fiber such as completely fluorinated polymer or polymethylmethacrylate (PMMA)). In this example, the spiral portion 142 is made of polypropylene.
   The number of cell aggregates 2 to be trapped in the spiral portion 142 is arbitrary. The number may be one or more. In this example, one cell aggregate 2 is trapped per turn of the spiral portion 142.
   According to this example, the suction tip 1h is provided with the spiral portion 142 whose tubular passage is formed into a spiral shape. According to this configuration, even when two or more cell aggregates 2 are sucked, it is not necessary to form the suction tip 110 described referring to FIG. 16 in such a manner that the length of the horizontal portion 141 is relatively long. The length of the tubular passage can be extended, while keeping the space small. Thus, it is possible to miniaturize the object observation device incorporated with the suction tip 1h in this example.
(9) In the embodiment (fifth embodiment), a motor mainly moves the main body portion up and down, and also moves the main body portion in front and rear directions and in left and right directions for calibrating an object observation device. Alternatively, in the invention, a motor may be controlled to move the main body portion freely in three directions i.e. front and rear directions, left and right directions, and up and down directions. According to this configuration, it is possible to dispose a suction port of a suction tip at a position above a cell aggregate (object) without moving a stage on which a Petri dish is placed. In this case, the cell aggregate is not moved accompanied by movement of the stage. This is advantageous in accurately grasping the position of the cell aggregate and in accurately sucking the cell aggregate.
(10) In the embodiment (fifth embodiment), an object observation device incorporated with the suction tip described in detail in the first embodiment is described. Alternatively, in the invention, any device is applicable, as far as an object observation device is incorporated with a suction tip, as described in detailed in the second to fourth embodiments, wherein the suction tip includes a distal end portion internally provided with a tubular passage serving as a suction path for sucking an object, the distal end portion disposed in a substantially vertical direction when in use and including a suction port for sucking the object, the suction port being an opening formed in one end of the tubular passage; and a trap portion formed downstream of the distal end portion in a suction direction, and configured to trap the object to be sucked through the suction port.
(11) In the embodiment (fifth embodiment), an illumination means may be mounted on the object observation device. An example of the illumination means to be mounted may be a light source disposed above a vessel to be placed on a stage by a certain distance, and configured to illuminate an object held in the vessel with illumination light from above. The light source is not specifically limited. It is possible to use a halogen lamp (6V30W), a tungsten lamp, a mercury lamp, a xenon lamp, or a light emitting diode (LED). Further, it is possible to provide a condenser incorporated with a collector lens, an aperture stop, and a condenser lens, in addition to the light source.
   Further, the inventive object observation device may be configured as a device constituting an illumination system and an imaging system of an inverted phase contrast microscope by providing a ring slit in a condenser, and by mounting a phase difference objective lens, a phase plate, and the like in an observing means (imaging device). Alternatively, it is possible to configure an object observation device provided with a configuration of observing an object (cell aggregate) using the principle of a fluorescent microscope by providing an excitation filter in a condenser and by providing a fluorescent filter in an imaging device. In this case, it is necessary to give fluorescence to a cell aggregate. In view of the above, when a non-fluorescent cell aggregate is observed, the cell aggregate is dyed with a fluorescent pigment such as trypan blue before measurement. The dyeing method is not specifically limited. Any preferred dyeing method may be used, as necessary. It is possible to use a chemically dyeing method or an antibody fluorescence dyeing method, for instance. In addition to the above, it is possible to use a method, in which a gene inducing a fluorescent protein such as GFP (Green Fluorescent Protein) is introduced to a cell aggregate by gene recombination for observation.
   Further, the inventive object observation device may be configured to observe a cell aggregate using the principle of a differential interference microscope by providing a polarizing plate and a Wollaston prism in a condenser. Furthermore, the inventive object observation device may be configured to observe a cell aggregate using the principles of a variety of microscopes such as a polarizing microscope, a stereoscopic microscope, a bright field microscope, a dark field microscope, an ultrasonic microscope, a confocal microscope, a laser scanning microscope, an electron microscope, a scanning probe microscope, an X-ray microscope, a virtual microscope, and a digital microscope.
(12) In the embodiment (fifth embodiment), a CCD image sensor is used as the imaging element. Alternatively, the inventive object observation device may use a variety of imaging elements such as a CMOS (Complementary Metal Oxide Semiconductor) image sensor.
(13) In the embodiment (fifth embodiment), an object observation device is configured such that one suction pipette is connected to a moving device (driving means). Alternatively, the inventive object observation device may be an object observation device configured such that two or more suction pipettes are connected to a moving device. This makes it possible to collect a plurality of cell aggregates at one time. This is advantageous in enhancing work efficiency.
(14) In the embodiment (fifth embodiment), a moving device is moved only up and down. Alternatively, the inventive object observation device may be provided with a moving device which is movable in an oblique direction in addition to three directions i.e. front and rear directions, left and right directions, and up and down directions. This eliminates the need of moving a vessel by moving a stage in front and rear directions and in left and right directions.
(15) In the embodiment (fifth embodiment), images (including moving images) of a cell aggregate in a Petri dish (vessel), a suction port of a suction tip, and a trap portion are displayed on a display device of an imaging device so that the user is allowed to operate the suction tip while checking the images. Alternatively, the inventive object observation device may be configured such that a mirror barrel is mounted on an imaging device so that the user is allowed to directly observe the positions of a cell aggregate in a vessel, a suction port of a suction tip, and a trap portion through the mirror barrel.
(16) In the embodiment (sixth embodiment), an object observing method includes a sucking step of sucking a cell aggregate using the object observation device described in detail in the fifth embodiment. Alternatively, in the invention, any object observing method may be performed, as far as the object observing method includes a sucking step of sucking a cell aggregate, using a suction tip as described in detail in the second to fourth embodiments, wherein the suction tip includes a distal end portion internally provided with a tubular passage serving as a suction path for sucking an object, the distal end portion disposed in a substantially vertical direction when in use and including a suction port for sucking the object, the suction port being an opening formed in one end of the tubular passage; and a trap portion formed downstream of the distal end portion in a suction direction, and configured to trap the object to be sucked through the suction port.
(17) In the foregoing embodiments, a process to be carried out by the user may be automatically carried out by a robot by controlling the robot using a software or the like in which the process contents are programmed in advance. For instance, it is possible to control a robot using a software, in which a process of determining a cell aggregate of a shape suitable for the purpose of use from among the cell aggregates sunk on a Petri dish, and of moving a suction pipette to such a position that a suction port is disposed above the target cell aggregate; a process of searching a cell aggregate trapped in a trap portion after sucking the cell aggregate; and a process of comparing the shapes of the cell aggregate before and after a suction operation to check whether the sucked cell aggregate is damaged, are programmed.

The aforementioned embodiments mainly include the invention having the following configuration.

A suction tip according to an aspect of the invention is provided with an internal tubular passage serving as a suction path for sucking an object, a distal end portion disposed in a substantially vertical direction when in use and including a suction port for sucking the object, the suction port being an opening formed in one end of the tubular passage; and a trap portion formed downstream of the distal end portion in a suction direction, and configured to trap the object to be sucked through the suction port.

In the suction tip of the invention, the suction port is formed in one end of the distal end portion disposed in a substantially vertical direction when in use. Therefore, for instance, it is possible to accurately suck the object held in a liquid stored in a vessel in a state that the suction port comes sufficiently close to the object. The sucked object is trapped in a stationary state in the trap portion. Therefore, the object is not discharged through the suction port even when the suction port is immersed in the liquid.

In the aforementioned configuration, preferably, the suction tip may be further provided with a main body portion formed downstream of the trap portion in the suction direction, wherein the trap portion traps the object that is sucked to the main body portion and travels upstream in the suction direction.

According to the aforementioned configuration, the trap portion can trap not only the object sucked to the trap portion but also the object that is sucked to the main body portion via the trap portion and travels upstream in the suction direction while sinking in the gravitational direction.

In the aforementioned configuration, preferably, the suction tip may be further provided with a converting portion continuously formed with the distal end portion, and configured to convert an extending direction of the tubular passage in the distal end portion, wherein the trap portion includes a horizontal portion formed downstream of the converting portion in the suction direction.

According to the aforementioned configuration, the object sucked through the tubular passage travels downstream in the suction direction via the converting portion, and is held horizontally in the horizontal portion. Therefore, when a flow of liquid flowing upstream in the suction direction is generated in order to eject the object, it is easy to flow the object together with the generated flow of liquid and to eject the object.

In the aforementioned configuration, preferably, the suction tip may be further provided with a converting portion continuously formed with the distal end portion, and configured to convert an extending direction of the tubular passage in the distal end portion downward with respect to a horizontal direction, wherein the trap portion includes a re-converting portion formed downstream of the converting portion in the suction direction, and configured to re-convert the extending direction of the tubular passage upward with respect to the horizontal direction for trapping the object.

According to the aforementioned configuration, the extending direction of the tubular passage is converted downward with respect to a horizontal direction by the converting portion. In view of the above, the tubular passage has a folded shape bulging upward by the converting portion when in use. Consequently, the object sucked via the converting portion cannot cross over the converting portion simply by falling in the gravitational direction, and is not ejected through the suction port. Further, the extending direction of the tubular passage is re-converted upward with respect to a horizontal direction by the re-converting portion. In view of the above, the re-converting portion has a folded shape bulging downward when in use. Thus, the tubular passage from the converting portion to the re-converting portion has a slope inclined downward. Consequently, not only the object that has reached the main body portion but also the object that has traveled at a position immediately in front of the re-converting portion via the converting portion are stably trapped, while falling in the re-converting portion. Thus, when the user checks the re-converting portion by an externally mounted microscope or the like, it is easy for the user to check the shape or the like of the object.

In the aforementioned configuration, preferably, the trap portion may be provided with a projection piece projecting from the inner wall of the tubular passage, and the projection piece may restrain the object traveling downstream in the suction direction via the projection piece from traveling upstream in the suction direction for trapping the object.

According to the aforementioned configuration, the object is blocked by the projection piece. Thus, traveling of the object upstream in the suction direction is securely restrained, and the object is trapped in the trap portion.

In the aforementioned configuration, preferably, the trap portion may include a reduced diameter portion whose diameter is smaller than a diameter of the suction port of the distal end portion.

According to the aforementioned configuration, during a suction operation, the object is allowed to pass the diameter reduced portion by deforming or expanding the diameter reduced portion. After the object has passed the diameter reduced portion, it is impossible to deform or expand the diameter reduced portion simply by falling of the object in the gravitational direction or by contact of the object with the diameter reduced portion. Therefore, the object is held downstream of the diameter reduced portion in the trap portion in the suction direction. Thus, when the user checks the held object by an externally mounted microscope or the like, it is easy for the user to check the shape or the like of the object.

Preferably, the reduced diameter portion may include a tapered portion for narrowing a flow channel of the tubular passage toward downstream in the suction direction; and an inverse tapered portion continuing from the tapered portion, and configured to expand the flow channel of the tubular passage toward downstream in the suction direction.

According to the aforementioned configuration, it is easy to guide the object downstream in the suction direction along the tapered portion to a through-hole formed in the downstream end of the tapered portion in the suction direction. The object passes through the through-hole while deforming or accompanied by deformation of the through-hole. After having passed through the through-hole, the object sinks in the tubular passage along the inverse tapered portion while falling in the gravitational direction, and is held in the inverse tapered portion at a position near the through-hole. Thus, when the user checks the held object by an externally mounted microscope or the like, it is easy for the user to check the shape or the like of the object.

In the aforementioned configuration, preferably, the object may be a cell derived from a living body.

Cells derived from a living body have a relatively large deviation in shape, and the size of the cells is very small. In the invention, a suction port is formed in the distal end portion of the suction tip. Therefore, it is easy to move the suction port to very small cells derived from a living body, and to accurately suck the cells. Further, it is possible to hold the sucked object in the trap portion. Therefore, the object is not discharged through the suction port even when the suction port is immersed in a liquid. This eliminates the need of taking out the suction port to such a height that it is possible to judge whether the suction operation is successful. Furthermore, the sucked cell is trapped in the trap portion. Thus, when the user observes the trap portion by an externally mounted microscope or the like, it is easy for the user to check the shape or the like of the cells.. This is advantageous in contributing to enhanced work efficiency in the bio-related technology or in the pharmaceutical field.

In the aforementioned configuration, preferably, the object may be a cell aggregate derived from a living body.

Use of a cell aggregate derived from a living body is advantageous, as compared with a test result obtained by using one cell, in that an environment similar to the environment in the living body is re-configured within the cell aggregate, taking into consideration the interaction between the cells. It is possible to obtain a result taking into consideration the functions of the individual cells, and to adjust the experiment condition to be in conformity with a condition suitable for the environment in the living body. In view of the above, use of a cell aggregate is important in the regenerative medical field and in the field of developing pharmaceuticals such as anticancer agents. Further, cell aggregates have different shapes from each other. In the invention, a suction port is formed in the distal end portion of the suction tip. Therefore, it is easy to move the suction port to a very small cell aggregate derived from a living body, and to accurately suck the cell aggregate. Further, it is possible to hold the sucked object in the trap portion. Therefore, the object is not discharged through the suction port even when the suction port is immersed in a liquid. This eliminates the need of taking out the suction port to such a height that it is possible to judge whether the suction operation is successful. Furthermore, the sucked cell aggregate is trapped in the trap portion. Thus, when the user observes the trap portion by an externally mounted microscope or the like, it is easy for the user to check the shape or the like of the cell aggregate. This is advantageous in contributing to enhanced work efficiency in the bio-related technology or in the pharmaceutical field.

An object observation device according to yet another aspect of the invention is provided with a vessel including an inner bottom portion, and configured to store a liquid containing an object to be sucked; a suction tip including an internal tubular passage serving as a suction path for sucking the object, a distal end portion disposed in a substantially vertical direction when in use and including a suction port for sucking the object, the suction port being an opening formed in one end of the tubular passage, and a trap portion formed downstream of the distal end portion in a suction direction, and configured to trap the object to be sucked through the suction port; a suction means connected to the suction tip, and configured to generate a suction force for sucking the object; and an observing means provided with an optical lens system for capturing an image of the object to be trapped in the trap portion within a depth of field of the optical lens system.

A distal end of the suction means for use in the object observation device of the invention is connected to the suction tip. The suction tip includes the suction port formed in one end of the distal end portion disposed in a substantially vertical direction when in use. Therefore, for instance, it is possible to move the suction port sufficiently close to the object held in a liquid stored in a vessel in a substantially vertical direction for sucking the object. The sucked object is trapped in a stationary state in the trap portion. Therefore, the object is not discharged through the suction port even when the suction port is immersed in the liquid. Further, an image of the object trapped in the trap portion is captured within the depth of field of the optical lens system provided in the observing means. Therefore, it is easy for the user to check whether the object is trapped in the trap portion.

In the aforementioned configuration, preferably, the optical lens system of the observing means may be an optical lens system for capturing the image of the object held in the vessel within the depth of field of the optical lens system.

According to the aforementioned configuration, not only an image of the object trapped in the trap portion but also an image of the object held in the vessel are captured within the depth of field. This allows for the user to sequentially check the position and the shape of the object before a suction operation, and to check the position and the shape of the object after the suction operation. This is advantageous in enhancing work efficiency.

An object observing method according to still another object of the invention includes a sucking step of sucking an object by a suction means connected to a suction tip, the suction tip provided with an internal tubular passage serving as a suction path for sucking the object, a distal end portion disposed in a substantially vertical direction when in use and including a suction port for sucking the object, the suction port being an opening formed in one end of the tubular passage, and a trap portion formed downstream of the distal end portion in a suction direction, and configured to trap the object to be sucked through the suction port; a trapping step of trapping the sucked object in the trap portion; and an observing step of observing the trapped object.

In the object observing method of the invention, the trapping step of trapping the sucked object in the trap portion in a stationary state follows the sucking step of sucking the object. Therefore, the sucked object is not discharged through the suction port, while sinking in the gravitational direction, even when the suction port is immersed in a liquid. Further, the object observing method includes the observing step of observing the object trapped in the trap portion. Therefore, it is possible to check the shape of the object, in addition to judgment as to whether the suction operation of the object is successful.

## Claims

1. A suction tip, comprising:
an internal tubular passage serving as a suction path for sucking an object,
a distal end portion disposed in a substantially vertical direction when in use and including a suction port for sucking the object, the suction port being an opening formed in one end of the tubular passage; and
a trap portion formed downstream of the distal end portion in a suction direction, and configured to trap the object to be sucked through the suction port.

2. The suction tip according to Claim 1, further comprising:
a main body portion formed downstream of the trap portion in the suction direction, wherein
the trap portion traps the object that is sucked to the main body portion and travels upstream in the suction direction.

3. The suction tip according to Claim 1 or 2, further comprising:
a converting portion continuously formed with the distal end portion, and configured to convert an extending direction of the tubular passage in the distal end portion, wherein
the trap portion includes a horizontal portion formed downstream of the converting portion in the suction direction.

4. The suction tip according to claim 1 or 2, further comprising:
a converting portion continuously formed with the distal end portion, and configured to convert an extending direction of the tubular passage in the distal end portion downward with respect to a horizontal direction, wherein
the trap portion includes a re-converting portion formed downstream of the converting portion in the suction direction, and configured to re-convert the extending direction of the tubular passage upward with respect to the horizontal direction for trapping the object.

5. The suction tip according to any one of Claims 1 to 4, wherein
the trap portion is provided with a projection piece projecting from an inner wall of the tubular passage, and
the projection piece restrains the object traveling downstream in the suction direction via the projection piece from traveling upstream in the suction direction for trapping the object.

6. The suction tip according to Claim 1, wherein
the trap portion includes a reduced diameter portion whose diameter is smaller than a diameter of the suction port of the distal end portion.

7. The suction tip according to Claim 6, wherein
the reduced diameter portion includes
a tapered portion for narrowing a flow channel of the tubular passage toward downstream in the suction direction; and
an inverse tapered portion continuing from the tapered portion and configured to expand the flow channel of the tubular passage toward downstream in the suction direction.

8. The suction tip according to any one of Claims 1 to 7, wherein
the object is a cell derived from a living body.

9. The suction tip according to Claim 8, wherein
the object is a cell aggregate derived from a living body.

10. An object observation device, comprising:
a vessel including an inner bottom portion, and configured to store a liquid containing an object to be sucked;
a suction tip including
an internal tubular passage serving as a suction path for sucking the object,
a distal end portion disposed in a substantially vertical direction when in use and including a suction port for sucking the object, the suction port being an opening formed in one end of the tubular passage, and
a trap portion formed downstream of the distal end portion in a suction direction, and configured to trap the object to be sucked through the suction port;
a suction means connected to the suction tip, and configured to generate a suction force for sucking the object; and
an observing means provided with an optical lens system for capturing an image of the object to be trapped in the trap portion within a depth of field of the optical lens system.

11. The object observation device according to Claim 10, wherein
the optical lens system of the observing means is an optical lens system for capturing the image of the object held in the vessel within the depth of field of the optical lens system.

12. An object observing method, comprising:
a sucking step of sucking an object by a suction means connected to a suction tip, the suction tip including
an internal tubular passage serving as a suction path for sucking the object, a distal end portion disposed in a substantially vertical direction when in use and including a suction port for sucking the object, the suction port being an opening formed in one end of the tubular passage, and
a trap portion formed downstream of the distal end portion in a suction direction, and configured to trap the object to be sucked through the suction port;
a trapping step of trapping the sucked object in the trap portion; and
an observing step of observing the trapped object.
